# EUROPEAN PATENT APPLICATION

(11) **EP 2 730 573 A1**
(43) Date of publication of application: **14.05.2014**
(21) Application number: 12807591.8
(22) Date of filing: 04.07.2012
(51) Int. Cl.: C07D 413/14, A61K 31/454, A61K 31/4545, A61K 31/506, A61P 1/00, A61P 5/00, A61P 9/12, A61P 25/04, A61P 25/08, A61P 25/16, A61P 25/20, A61P 25/22, A61P 25/24, A61P 25/28, A61P 25/30, A61P 29/00, A61P 37/00, A61P 43/00, C07D 498/04

(54) **METHYLPIPERIDINE DERIVATIVE**

(30) Priority: 05.07.2011 JP 2011149336
(71) Applicant: Taisho Pharmaceutical Co., Ltd., Tokyo 170-8633 (JP)
(72) Inventor: ABE, Masahito, Tokyo 170-8633 (JP); FUTAMURA, Aya, Tokyo 170-8633 (JP); SUZUKI, Ryo, Tokyo 170-8633 (JP); NOZAWA, Dai, Tokyo 170-8633 (JP); OHTA, Hiroshi, Tokyo 170-8633 (JP); ARAKI, Yuko, Tokyo 170-8633 (JP)
(74) Representative: HOFFMANN EITLE
(86) International application number: PCT/JP2012/067038
(87) International publication number: WO 2013/005755

(57) **Abstract**

A compound represented by formula (IA) or a pharmaceutically acceptable salt thereof, which acts relying on an orexin (OX) receptor antagonistic activity and is useful for the treatment or prevention of diseases such as sleep disorder, depression, anxiety disorder, panic disorder, schizophrenia, drug dependence, Alzheimer's disease, Parkinson's disease, Huntington's chorea, eating disorder, head ache, migraine, pain, gastrointestinal diseases, epilepsy, inflammations, immune-related diseases, endocrine-related diseases and hypertension.

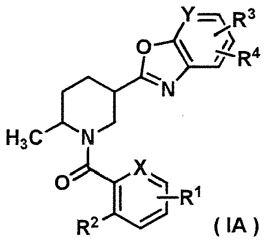

## Description

### Technical Field

The present invention relates to a compound having orexin (OX) receptor antagonist activity and a pharmaceutically acceptable salt thereof, and a therapeutic or prophylactic agent for diseases such as sleep disorder, depression, anxiety disorder, panic disorder, schizophrenia, drug dependence, Alzheimer's disease, Parkinson's disease, Huntington's chorea, eating disorder, headache, migraine, pain, gastrointestinal disease, epilepsy, inflammation, immune-related disease, endocrine-related disease, and hypertension, comprising the same as an active ingredient.

### Background Art

Orexin is a neuropeptide that is spliced from prepro-orexin specifically expressed in the lateral hypothalamic area. OX-A composed of 33 amino acids and OX-B composed of 28 amino acids have been identified so far. Both of them are deeply involved in the regulation of sleep-wake patterns or the regulation of feeding behavior.

OX-A and OX-B both act on OX receptors. Two subtypes of OX receptors, OX1 and OX2 receptors, have been cloned so far. Both of these receptors are known as seven-transmembrane G protein-coupled receptors predominantly expressed in the brain. The OX1 receptor is specifically coupled with Gq among G protein subclasses, while the OX2 receptor is coupled with Gq and Gi/o (see Non Patent Literatures 1 and 2).

The OX receptors differ in tissue distribution depending on their subtypes. The OX1 receptor is highly densely expressed in the locus coeruleus which is the nucleus of origin of noradrenergic neurons, while the OX2 receptor is highly densely expressed in the tuberomammillary nucleus which is the nucleus of origin of histaminergic neurons (see Non Patent Literatures 3, 4, and 5). The expression of both the OX1 receptor and the OX2 receptor is seen in the raphe nucleus which is the nucleus of origin of serotoninergic neurons and the ventral tegmental area which is the nucleus of origin of dopaminergic neurons (see Non Patent Literature 3). Orexin neurons are projected into the brain stem, the hypothalamus, and the monoamine nerve system and provide excitatory input to their neurons. In addition, OX2 receptor expression is also seen in brain stem acetylcholine neurons involved in the regulation of REM sleep and also influences the activity of these nerve nuclei (see Non Patent Literatures 3 and 4).

In recent years, the OX1 and OX2 receptors have received attention in terms of their relation to sleep-wake regulation, and the usefulness of compounds having OX receptor antagonist activity has been studied. The administration of OX-A into the rat ventricle is found to increase motor activity (see Non Patent Literatures 6 and 7), increase stereotypical behavior (see Non Patent Literature 7), and prolong waking hours (see Non Patent Literature 6), for example. The effect of shortening the hours of REM sleep by the administration of OX-A is completely antagonized by pretreatment with OX receptor antagonists (see Non Patent Literature 8). Moreover, the administration of orally administrable substances that dually antagonize OX1 and OX2 receptors reportedly decreases physical activity, shortens sleep latency time, and increases the amounts of non-REM sleep and REM sleep (see Non Patent Literatures 9 and 10).

Compounds having various structures, for example, reviewed in Non Patent Literature 11, are known as compounds having OX receptor antagonist activity. Also, Patent Literatures 1 to 3 disclose substituted piperidine derivatives, but do not disclose a compound containing a benzoxazole ring directly bonded to a piperidine ring as described in the present application.

### Citation List

### Patent Literature

[Patent Literature 1] WO2008/147518
[Patent Literature 2] WO2010/048010
[Patent Literature 3] WO2010/048012

### Non Patent Literature

[Non Patent Literature 1] Zhu Y et al., J. Pharmacol. Sci., 92, 259-266, 2003.
[Non Patent Literature 2] Zeitzer JM et al., Trends Pharmacol. Sci., 27, 368-374, 2006.
[Non Patent Literature 3] Marcus JN et al., J. Comp. Neurol, 435, 6-25, 2001.
[Non Patent Literature 4] Trivedi JP et al., FEBS Lett, 438, 71-75, 1998.
[Non Patent Literature 5] Yamanaka A et al., Biochem. Biophys. Res. Commun., 290, 1237-1245, 2002.
[Non Patent Literature 6] Hagan JJ et al., Proc. Natl. Acad. Sci. USA, 96, 10911-10916, 1999.
[Non Patent Literature 7] Nakamura T et al., Brain Res., 873, 181-187, 2000.
[Non Patent Literature 8] Smith MI et al., Neurosci. Lett., 341, 256-258, 2003.
[Non Patent Literature 9] Brisbare-Roch C et al., Nat. Med., 13, 150-155, 2007.
[Non Patent Literature 10] Cox CD et al., J. Med. Chem., 53, 5320-5332, 2010.
[Non Patent Literature 11] John G et al., ChemMedChem., 5, 1197-1214, 2010.

### Summary of Invention

### Technical Problem

An object of the present invention is to find a novel compound having OX receptor antagonist activity and to provide a therapeutic or prophylactic agent for diseases such as sleep disorder, depression, anxiety disorder, panic disorder, schizophrenia, drug dependence, Alzheimer's disease, Parkinson's disease, Huntington's chorea, eating disorder, headache, migraine, pain, gastrointestinal disease, epilepsy, inflammation, immune-related disease, endocrine-related disease, and hypertension. More specifically, an object of the present invention is to provide a novel compound that exhibits excellent OX receptor antagonist activity and also exhibits excellent pharmacokinetics and safety.

### Solution to Problem

The present inventors have conducted diligent studies on a compound of a novel skeleton having antagonistic action on orexin receptors and consequently completed the present invention by finding that a certain methylpiperidine derivative represented by the formula shown below has excellent OX receptor antagonist activity.

Hereinafter, the present invention will be described in detail. The aspect of the present invention (hereinafter, referred to as the "compound of the present invention") is as shown below.
(1) A methylpiperidine derivative represented by formula (IA): 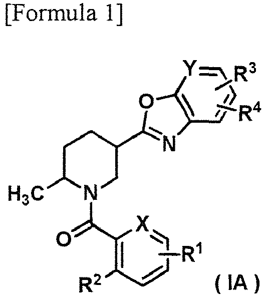 wherein
   X represents a nitrogen atom or a formula CH;
   Y represents a nitrogen atom or a formula CH;
   R¹ represents a hydrogen atom, a halogen atom, a cyano group, a C₁₋₆ alkyl group (wherein the C₁₋₆ alkyl group may be substituted with 1 to 3 halogen atoms), or a C₁₋₆ alkoxy group;
   R² represents a heteroaryl group (wherein the heteroaryl group may be substituted with 1 to 3 identical or different substituents selected from the group consisting of a halogen atom and a C₁₋₆ alkoxy group); and
   R³ and R⁴ are the same or different and each represent a hydrogen atom, a halogen atom, a cyano group, a C₁₋₆ alkyl group, or a C₁₋₆ alkoxy group (wherein the C₁₋₆ alkyl group and the C₁₋₆ alkoxy group may each be substituted with 1 to 3 halogen atoms)
   or a pharmaceutically acceptable salt thereof.
(2) The methylpiperidine derivative or the pharmaceutically acceptable salt thereof according to (1), wherein in formula (IA),
   R³ is a halogen atom, a cyano group, a C₁₋₆ alkyl group, or a C₁₋₆ alkoxy group (wherein the C₁₋₆ alkyl group and the C₁₋₆ alkoxy group may each be substituted with 1 to 3 halogen atoms), and
   R⁴ is a hydrogen atom or a halogen atom.
(3) The methylpiperidine derivative or the pharmaceutically acceptable salt thereof according to (1) or (2), wherein in formula (IA),
   R² is a triazolyl group or a pyrimidinyl group (wherein the pyrimidinyl group may be substituted with 1 to 3 identical or different substituents selected from the group consisting of a halogen atom and a C₁₋₆ alkoxy group).
(4) The methylpiperidine derivative or the pharmaceutically acceptable salt thereof according to any one of (1) to (3), wherein in formula (IA),
   R¹ is a hydrogen atom, a halogen atom, or a C₁₋₆ alkyl group.
(5) The methylpiperidine derivative or the pharmaceutically acceptable salt thereof according to any one of (1) to (4), wherein in formula (IA),
   Y is a formula CH, and
   R² is a triazolyl group or a pyrimidinyl group.
(6) The methylpiperidine derivative or the pharmaceutically acceptable salt thereof according to any one of (1) to (5), wherein in formula (IA),
   X is a nitrogen atom, and
   R⁴ is a hydrogen atom.
(7) The methylpiperidine derivative or the pharmaceutically acceptable salt thereof according to any one of (1) to (6), wherein formula (IA) is formula (IIA): 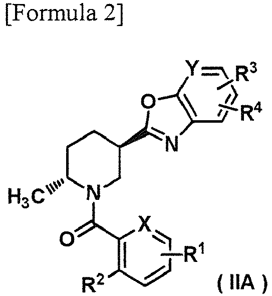
(8) A methylpiperidine derivative represented by formula (I): 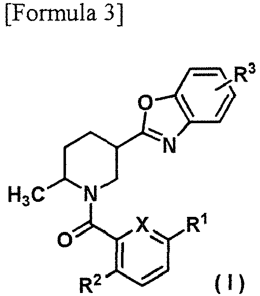 wherein
   X represents a nitrogen atom or a formula CH;
   R¹ represents a hydrogen atom, a halogen atom, or a C₁₋₆ alkyl group;
   R² represents a heteroaryl group (wherein the heteroaryl group may be substituted with 1 to 3 halogen atoms); and
   R³ represents a hydrogen atom, a halogen atom, a C₁₋₆ alkyl group, or a C₁₋₆ alkoxy group (wherein the C₁₋₆ alkyl group and the C₁₋₆ alkoxy group may each be substituted with 1 to 3 halogen atoms)
   or a pharmaceutically acceptable salt thereof.
(9) The methylpiperidine derivative or the pharmaceutically acceptable salt thereof according to (8), wherein in formula (I),
   X is a nitrogen atom,
   R¹ is a hydrogen atom or a C₁₋₆ alkyl group,
   R² is a triazolyl group or a pyrimidinyl group, and
   R³ is a halogen atom.
(10) The methylpiperidine derivative or the pharmaceutically acceptable salt thereof according to (8) or (9), wherein formula (I) is formula (II): 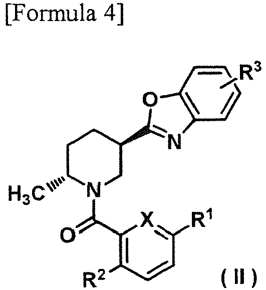
(11) A pharmaceutical composition comprising the methylpiperidine derivative or the pharmaceutically acceptable salt thereof according to any one of (1) to (10) as an active ingredient.
(12) A therapeutic or prophylactic agent for a disease such as sleep disorder, depression, anxiety disorder, panic disorder, schizophrenia, drug dependence, Alzheimer's disease, Parkinson's disease, Huntington's chorea, eating disorder, headache, migraine, pain, gastrointestinal disease, epilepsy, inflammation, immune-related disease, endocrine-related disease, or hypertension, comprising the methylpiperidine derivative or the pharmaceutically acceptable salt thereof according to any one of (1) to (10) as an active ingredient.

### Advantageous Effects of Invention

The methylpiperidine derivative of the present invention was shown to exhibit affinity for OX receptors and also exhibit antagonistic action on the stimulation of the receptors by physiological ligands.

### Description of Embodiments

Terms used herein are defined as follows:
The "halogen atom" means a fluorine atom, a chlorine atom, a bromine atom, and an iodine atom.
The "C₁₋₆ alkyl group" means linear or branched alkyl groups having 1 to 6 carbon atoms. Examples thereof include methyl, ethyl, n-propyl, isopropyl, n-butyl, isobutyl, sec-butyl, tert-butyl, n-pentyl, isopentyl, neopentyl, tert-pentyl, 1-ethylpropyl, n-hexyl, isohexyl, and neohexyl groups.
The "C₁₋₆ alkoxy group" means linear or branched alkoxy groups having 1 to 6 carbon atoms. Examples thereof include methoxy, ethoxy, n-propoxy, isopropoxy, n-butoxy, isobutoxy, sec-butoxy, tert-butoxy, n-pentyloxy, isopentyloxy, neopentyloxy, tert-pentyloxy, 1-ethylpropoxy, and n-hexyloxy groups.
The "heteroaryl" means 5-membered or 6-membered heteroaryl composed of 1 to 5 carbon atoms and one or more identical or different heteroatoms selected from the group consisting of nitrogen, oxygen and sulfur atoms. Examples thereof include pyrazolyl, imidazolyl, triazolyl, oxazolyl, thiazolyl, pyridyl, pyrazinyl, pyrimidinyl, and pyridazinyl groups.

The "sleep disorder" described herein refers to disorder associated with the onset of sleep, sleep phase duration, or wakefulness. Examples thereof include insomnia. Examples of insomnia classification include sleep-onset disorder, arousal during sleep, early-morning awakening, and deep-sleep disorder.

The "pharmaceutically acceptable salt" described herein means pharmaceutically acceptable acid-addition salts. The acid used includes: salts with inorganic acids such as sulfuric acid, hydrochloric acid, hydrobromic acid, phosphoric acid, and nitric acid; and salts with organic acids such as acetic acid, benzoic acid, oxalic acid, lactic acid, malic acid, tartaric acid, fumaric acid, maleic acid, citric acid, malonic acid, mandelic acid, gluconic acid, galactaric acid, glucoheptonic acid, glycolic acid, glutamic acid, methanesulfonic acid, ethanesulfonic acid, benzenesulfonic acid, p-toluenesulfonic acid, camphorsulfonic acid, and naphthalene-2-sulfonic acid. The salt can be converted from a free form by a conventional method.

Preferred forms of the compound of the present invention will be shown below.

A compound wherein Y is a formula CH is preferred.

A compound wherein X is a nitrogen atom is preferred.

A compound wherein R¹ is a hydrogen atom, a halogen atom (except for an iodine atom), or a C₁₋₆ alkyl group is preferred. In this context, a compound in which the halogen atom is a fluorine atom is more preferred. Also, a compound wherein the C₁₋₆ alkyl group is a methyl group is more preferred.

A compound wherein R² is a triazolyl group or a pyrimidinyl group is preferred. A compound wherein R² is a 1,2,3-triazol-2-yl group or a pyrimidin-2-yl group is more preferred.

A compound wherein R³ is a halogen atom, a cyano group, a C₁₋₆ alkyl group, or a C₁₋₆ alkoxy group (wherein the C₁₋₆ alkyl group and the C₁₋₆ alkoxy group may each be substituted with 1 to 3 halogen atoms) is preferred. A compound wherein R³ is a halogen atom or a C₁₋₆ alkyl group (wherein the C₁₋₆ alkyl group is substituted with 1 to 3 fluorine atoms) is more preferred. A compound wherein R³ is a fluorine atom, a chlorine atom, or a trifluoromethyl group is further preferred. A compound wherein R³ is a fluorine atom or a chlorine atom is particularly preferred.

A compound wherein R⁴ is a hydrogen atom or a halogen atom is preferred. A compound wherein R⁴ is a hydrogen atom or a fluorine atom is more preferred.

Examples of preferred compounds among these compounds of the present invention include
[(2R,5R)-5-(5-chloro-1,3-benzoxazol-2-yl)-2-methylpiperidin-1-yl][6-methyl-3-(2H-1,2,3-triazol-2-yl)pyridin-2-yl]methanone,
[(2R*,5R*)-5-(5-chloro-1,3-benzoxazol-2-yl)-2-methylpiperidin-1-yl][5-fluoro-2-(2H-1,2,3-triazol-2-yl)phenyl]methanone,
[(2R*,5R*)-5-(5-chloro-1,3-benzoxazol-2-yl)-2-methylpiperidin-1-yl][2-(2H-1,2,3-triazol-2-yl)phenyl]methanone,
[(2R*,5R*)-5-(5-chloro-1,3-benzoxazol-2-yl)-2-methylpiperidin-1-yl][6-methyl-3-(pyrimidin-2-yl)pyridin-2-yl]methanone,
[(2R*,5R*)-5-(5-chloro-1,3-benzoxazol-2-yl)-2-methylpiperidin-1-yl][5-methyl-2-(pyrimidin-2-yl)phenyl]methanone,
[(2R*,5R*)-5-(5-chloro-1,3-benzoxazol-2-yl)-2-methylpiperidin-1-yl][5-fluoro-2-(pyrimidin-2-yl)phenyl]methanone,
[(2R*,5R*)-5-(5-chloro-1,3-benzoxazol-2-yl)-2-methylpiperidin-1-yl][2-(pyrimidin-2-yl)phenyl]methanone,
[(2R*,5R*)-5-(5-fluoro-1,3-benzoxazol-2-yl)-2-methylpiperidin-1-yl][5-methyl-2-(pyrimidin-2-yl)phenyl]methanone,
[(2R*,5R*)-5-(5-chloro-1,3-benzoxazol-2-yl)-2-methylpiperidin-1-yl][5-methyl-2-(2H-1,2,3-triazol-2-yl)phenyl]methanone,
[(2R*,5R*)-5-(5-fluoro-1,3-benzoxazol-2-yl)-2-methylpiperidin-1-yl][5-methyl-2-(2H-1,2,3-triazol-2-yl)phenyl]methanone,
[(2R*,5R*)-5-(5-fluoro-1,3-benzoxazol-2-yl)-2-methylpiperidin-1-yl][6-methyl-3-(2H-1,2,3-triazol-2-yl)pyridin-2-yl]methanone,
[(2R,5R)-5-(5-chloro-1,3-benzoxazol-2-yl)-2-methylpiperidin-1-yl][5-chloro-2-(pyrimidin-2-yl)phenyl]methanone,
[(2R,5R)-5-(5-chloro-1,3-benzoxazol-2-yl)-2-methylpiperidin-1-yl][4-fluoro-2-(2H-1,2,3-triazol-2-yl)phenyl]methanone,
[(2R,5R)-5-(5-chloro-1,3-benzoxazol-2-yl)-2-methylpiperidin-1-yl][3-fluoro-2-(2H-1,2,3-triazol-2-yl)phenyl]methanone,
[(2R,5R)-5-(5-chloro-1,3-benzoxazol-2-yl)-2-methylpiperidin-1-yl][2-fluoro-6-(2H-1,2,3-triazol-2-yl)phenyl]methanone,
[(2R,5R)-5-(5-chloro-1,3-benzoxazol-2-yl)-2-methylpiperidin-1-yl][4-fluoro-2-(pyrimidin-2-yl)phenyl]methanone,
[(2R,5R)-5-(5-chloro-1,3-benzoxazol-2-yl)-2-methylpiperidin-1-yl][2-fluoro-6-(pyrimidin-2-yl)phenyl]methanone,
[5-bromo-2-(2H-1,2,3-triazol-2-yl)phenyl][(2R,5R)-5-(5-chloro-1,3-benzoxazol-2-yl)-2-methylpiperidin-1-yl]methanone,
[(2R,5R)-5-(5-chloro-1,3-benzoxazol-2-yl)-2-methylpiperidin-1-yl][5-fluoro-2-(5-methoxypyrimidin-2-yl)phenyl]methanone,
[(2R,5R)-5-(5-chloro-1,3-benzoxazol-2-yl)-2-methylpiperidin-1-yl][4-methyl-2-(2H-1,2,3-triazol-2-yl)phenyl]methanone,
[(2R,5R)-5-(5-fluoro-1,3-benzoxazol-2-yl)-2-methylpiperidin-1-yl][2-(2H-1,2,3-triazol-2-yl)phenyl]methanone,
[(2R,5R)-5-(5-fluoro-1,3-benzoxazol-2-yl)-2-methylpiperidin-1-yl][5-fluoro-2-(2H-1,2,3-triazol-2-yl)phenyl]methanone,
[5-chloro-2-(2H-1,2,3-triazol-2-yl)phenyl][(2R,5R)-5-(5-fluoro-1,3-benzoxazol-2-yl)-2-methylpiperidin-1-yl]methanone,
[(2R,5R)-5-(5-fluoro-1,3-benzoxazol-2-yl)-2-methylpiperidin-1-yl][2-(pyrimidin-2-yl)phenyl]methanone,
[(2R,5R)-5-(5-fluoro-1,3-benzoxazol-2-yl)-2-methylpiperidin-1-yl][5-fluoro-2-(pyrimidin-2-yl)phenyl]methanone,
[5-chloro-2-(pyrimidin-2-yl)phenyl][(2R,5R)-5-(5-fluoro-1,3-benzoxazol-2-yl)-2-methylpiperidin-1-yl]methanone,
[(2R,5R)-5-(5-fluoro-1,3-benzoxazol-2-yl)-2-methylpiperidin-1-yl][4-fluoro-2-(2H-1,2,3-triazol-2-yl)phenyl]methanone,
[(2R,5R)-5-(5-fluoro-1,3-benzoxazol-2-yl)-2-methylpiperidin-1-yl][2-fluoro-6-(2H-1,2,3-triazol-2-yl)phenyl]methanone,
[5-methyl-2-(2H-1,2,3-triazol-2-yl)phenyl]{(2R,5R)-2-methyl-5-[5-(trifluoromethyl)-1,3-benzoxazol-2-yl]piperidin-1-yl}methanone,
[5-methyl-2-(pyrimidin-2-yl)phenyl]{(2R,5R)-2-methyl-5-[5-(trifluoromethyl)-1,3-benzoxazol-2-yl]piperidin-1-yl} methanone,
[5-chloro-2-(pyrimidin-2-yl)phenyl]{(2R,5R)-2-methyl-5-[5-(trifluoromethyl)-1,3-benzoxazol-2-yl]piperidin-1-yl}methanone,
[(2R,5R)-5-(6-chloro-1,3-benzoxazol-2-yl)-2-methylpiperidin-1-yl][5-methyl-2-(2H-1,2,3-triazol-2-yl)phenyl]methanone,
[(2R,5R)-5-(6-chloro-1,3-benzoxazol-2-yl)-2-methylpiperidin-1-yl][5-methyl-2-(pyrimidin-2-yl)phenyl]methanone,
[(2R,5R)-5-(6-chloro-1,3-benzoxazol-2-yl)-2-methylpiperidin-1-yl][5-fluoro-2-(pyrimidin-2-yl)phenyl]methanone,
[(2R,5R)-5-(6-chloro-1,3-benzoxazol-2-yl)-2-methylpiperidin-1-yl][6-methyl-3-(2H-1,2,3-triazol-2-yl)pyridin-2-yl]methanone,
[(2R,5R)-5-(6-chloro-1,3-benzoxazol-2-yl)-2-methylpiperidin-1-yl][4-fluoro-2-(2H-1,2,3-triazol-2-yl)phenyl]methanone,
[(2R,5R)-5-(6-chloro-1,3-benzoxazol-2-yl)-2-methylpiperidin-1-yl][2-fluoro-6-(2H-1,2,3-triazol-2-yl)phenyl]methanone,
[(2R,5R)-5-(5,6-difluoro-1,3-benzoxazol-2-yl)-2-methylpiperidin-1-yl][5-methyl-2-(2H-1,2,3-triazol-2-yl)phenyl]methanone,
[(2R,5R)-5-(5,6-difluoro-1,3-benzoxazol-2-yl)-2-methylpiperidin-1-yl][5-methyl-2-(pyrimidin-2-yl)phenyl]methanone,
[(2R,5R)-5-(5,6-difluoro-1,3-benzoxazol-2-yl)-2-methylpiperidin-1-yl][5-fluoro-2-(pyrimidin-2-yl)phenyl]methanone,
[5-chloro-2-(pyrimidin-2-yl)phenyl][(2R,5R)-5-(5,6-difluoro-1,3-benzoxazol-2-yl)-2-methylpiperidin-1-yl]methanone,
[(2R, 5R)-5-(5,6-difluoro-1,3-benzoxazol-2-yl)-2-methylpiperidin-1-yl][6-methyl-3-(2H-1,2,3-triazol-2-yl)pyridin-2-yl]methanone,
[(2R,5R)-5-(5,6-difluoro-1,3-benzoxazol-2-yl)-2-methylpiperidin-1-yl][4-fluoro-2-(2H-1,2,3-triazol-2-yl)phenyl]methanone,
2-{((3R,6R)-6-methyl-1-[5-methyl-2-(2H-1,2,3-triazol-2-yl)benzoyl]piperidin-3-yl}-1,3-benzoxazole-5-carbonitrile,
2-{((3R,6R)-6-methyl-1-[5-methyl-2-(pyrimidin-2-yl)benzoyl]piperidin-3-yl}-1,3-benzoxazole-5-carbonitrile,
[(2R,5R)-5-(6,7-difluoro-1,3-benzoxazol-2-yl)-2-methylpiperidin-1-yl][5-methyl-2-(2H-1,2,3-triazol-2-yl)phenyl]methanone,
[(2R,5R)-5-(6,7-difluoro-1,3 -benzoxazol-2-yl)-2-methylpiperidin-1-yl][5-methyl-2-(pyrimidin-2-yl)phenyl] methanone,
[(2R,5R)-5-(4,6-difluoro-1,3-benzoxazol-2-yl)-2-methylpiperidin-1-yl][5-methyl-2-(2H-1,2,3-triazol-2-yl)phenyl]methanone,
[(2R,5R)-5-(4,6-difluoro-1,3-benzoxazol-2-yl)-2-methylpiperidin-1-yl][5-methyl-2-(pyrimidin-2-yl)phenyl]methanone,
[(2R,5R)-5-(5,7-difluoro-1,3-benzoxazol-2-yl)-2-methylpiperidin-1-yl][5-methyl-2-(2H-1,2,3-triazol-2-yl)phenyl]methanone,
[(2R,5R)-5-(5,7-difluoro-1,3-benzoxazol-2-yl)-2-methylpiperidin-1-yl][5-methyl-2-(pyrimidin-2-yl)phenyl]methanone,
[(2R,5R)-5-(7-fluoro-1,3-benzoxazol-2-yl)-2-methylpiperidin-1-yl][5-methyl-2-(2H-1,2,3-triazol-2-yl)phenyl]methanone,
[(2R,5R)-5-(4-fluoro-1,3-benzoxazol-2-yl)-2-methylpiperidin-1-yl][5-methyl-2-(2H-1,2,3-triazol-2-yl)phenyl]methanone,
[(2R,5R)-5-(4-fluoro-1,3-benzoxazol-2-yl)-2-methylpiperidin-1-yl][5-methyl-2-(pyrimidin-2-yl)phenyl]methanone,
[(2R,5R)-5-(4-chloro-1,3-benzoxazol-2-yl)-2-methylpiperidin-1-yl][5-methyl-2-(2H-1,2,3-triazol-2-yl)phenyl]methanone,
[(2R,5R)-5-(4-chloro-1,3-benzoxazol-2-yl)-2-methylpiperidin-1-yl][5-methyl-2-(pyrimidin-2-yl)phenyl]methanone,
[(2R,5R)-5-(4-chloro-1,3-benzoxazol-2-yl)-2-methylpiperidin-1-yl][5-fluoro-2-(pyrimidin-2-yl)phenyl]methanone,
[(2R,5R)-5-(6-chloro[1,3]oxazolo[5,4-b]pyridin-2-yl)-2-methylpiperidin-1-yl][5-methyl-2-(2H-1,2,3-triazol-2-yl)phenyl]methanone, and
[(2R,5R)-5-(6-chloro[1,3]oxazolo[5,4-b]pyridin-2-yl)-2-methylpiperidin-1-yl][4-fluoro-2-(2H-1,2,3-triazol-2-yl)phenyl]methanone.

The compound of the present invention may form a hydrate or a solvate. These compounds are also included in the scope of the present invention. Likewise, a pharmaceutically acceptable salt of the hydrate or the solvate of the compound of the present invention is also included in the scope of the present invention.

The compound of the present invention encompasses all of enantiomers, diastereomers, equilibrium compounds, and mixtures of these compounds at arbitrary ratios, racemates thereof, and the like.

The compound according to the present invention also encompasses compounds containing radioisotopes or stable isotopes replaced for one or more hydrogen atoms, carbon atoms, nitrogen atoms, oxygen atoms, or fluorine atoms. These labeled compounds are useful in, for example, metabolic or pharmacokinetic research or biological analysis as ligands or the like for receptors.

The compound according to the present invention can be administered orally or parenterally. Its dosage form includes tablets, capsules, granules, powders, dusts, troches, ointments, creams, skin patches, emulsions, suspensions, suppositories, injections, and the like. Any of these dosage forms can be produced by a routine formulation technique (e.g., a method prescribed by the Japanese Pharmacopoeia, 15the Edition). These dosage forms can be appropriately selected according to the symptoms, age, and body weight of a patient, and therapeutic purposes.

These preparations can be produced by mixing a composition containing the compound of the present invention with pharmacologically acceptable carriers, i.e., an excipient (e.g., crystalline cellulose, starch, lactose, and mannitol), a binder (e.g., hydroxypropylcellulose and polyvinylpyrrolidone), a lubricant (e.g., magnesium stearate and talc), and a disintegrant (e.g., carboxymethylcellulose calcium), and other various pharmacologically acceptable additives.

The compound of the present invention can be administered orally or parenterally to an adult patient at each dose of 0.001 to 500 mg once or several times a day. This dose can be appropriately increased or decreased depending on the type of a disease to be treated, the age, body weight, and symptoms of a patient, etc.

Examples of desirable profiles of the compound of the present invention include excellent drug efficacy, excellent disposition (favorable oral absorbability, etc.), excellent physical properties, and low toxicity.

Typical methods for producing the compound (IA) of the present invention will be shown below in Schemes A to C. The methods for producing the compound of the present invention shown below are provided for illustrative purposes and do not limit the scope of the present invention. In the examples of the production methods below, each compound may form a salt that does not hinder the reaction. 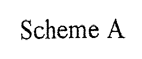 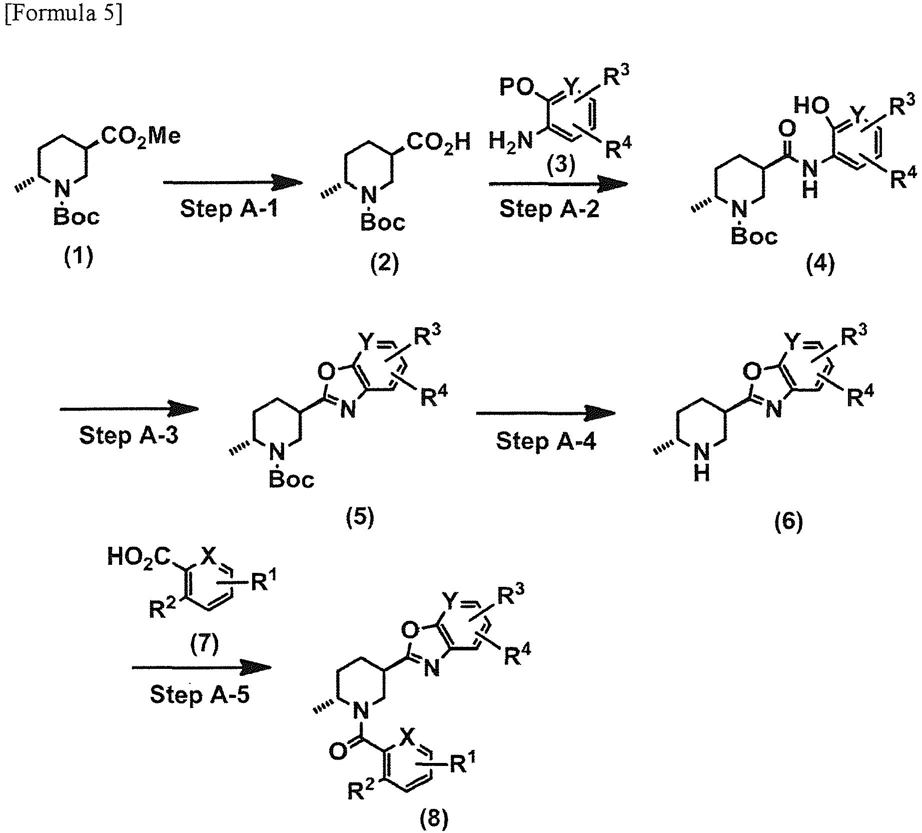 wherein Boc represents a tert-butoxycarbonyl group; X, Y, R¹, R², and R³ are as defined above; and P represents a general protective group for hydroxy groups, for example, a group described in J. F. W. McOmie, Protective Groups in Organic Chemistry and T. W. Greene and P. G. M. Wuts, Protective Groups in Organic Synthesis and specifically represents, for example, a triethylsilyl group.

Step A-1: The compound (2) can be obtained through the hydrolysis reaction of ester of a compound (1). The reaction in Step A-1 can be performed using an aqueous solution of a base such as lithium hydroxide, sodium hydroxide, or potassium hydroxide. A mixed solvent of methanol, ethanol, tetrahydrofuran, and the like may be used for increasing the solubility of a substrate or adjusting the reaction temperature. This reaction can be performed under conditions of 0°C to reflux temperature.
Step A-2: The compound (4) can be obtained through the condensation reaction of the compound (2) and a compound (3). Examples of the condensation reaction of Step A-2 include methods using dehydration-condensation agents. Examples of the dehydration-condensation agents include DMT-MM (4-(4,6-dimethoxy-1,3,5-triazin-2-yl)-4-methylmorpholinium chloride), 1-ethyl-3-(3-dimethylaminopropyl)carbodiimide hydrochloride, propane phosphonic acid anhydride, HATU [O-(7-azabenzotriazol-1-yl)-N,N,N',N'-tetramethyluronium hexafluorophosphate], TBTU (O-(benzotriazol-1-yl)-N,N,N,N'-tetramethyluronium tetrafluoroborate), dicyclohexylcarbodiimide, diphenylphosphonyl azide, and carbonyldiimidazole. If necessary, an activator such as 1-hydroxybenzotriazole or hydroxysuccinimide can be used. Examples of reaction solvents include N,N-dimethylformamide, tetrahydrofuran, dichloromethane, chloroform, toluene, ethyl acetate, methanol, ethanol, water, and mixed solvents thereof. This reaction can be performed using a base. Examples of the base include: organic amines such as pyridine, triethylamine, and diisopropylethylamine; and inorganic bases such as potassium carbonate. The reaction can be performed at 0°C to reflux temperature.
Step A-3: The compound (5) can be obtained from the compound (4) through ring-closing reaction. The reaction in Step A-3 can be performed under conditions of 0°C to reflux temperature using thionyl chloride, phosphorus oxychloride, polyphosphoric acid, acetic acid, triphenylphosphine/DEAD (diethyl azodicarboxylate), triphenylphosphine/hexachloroethane, or the like in the absence of a solvent or in a solvent such as acetonitrile, tetrahydrofuran, or chloroform or a mixed solvent thereof.
Step A-4: The compound (6) can be obtained through the reaction of the compound (5) with an acid such as hydrochloric acid, sulfuric acid, trifluoroacetic acid, p-toluenesulfonic acid, or methanesulfonic acid. The general overview of the reaction in Step A-4 can be found in J. F. W. McOmie, Protective Groups in Organic Chemistry and T. W. Greene and P. G. M. Wuts, Protective Groups in Organic Synthesis.
Step A-5: The compound (8) can be obtained through the condensation reaction of the compound (6) and a compound (7). The condensation reaction in Step A-5 can be performed according to the same reaction conditions as in Step A-2. 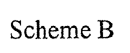 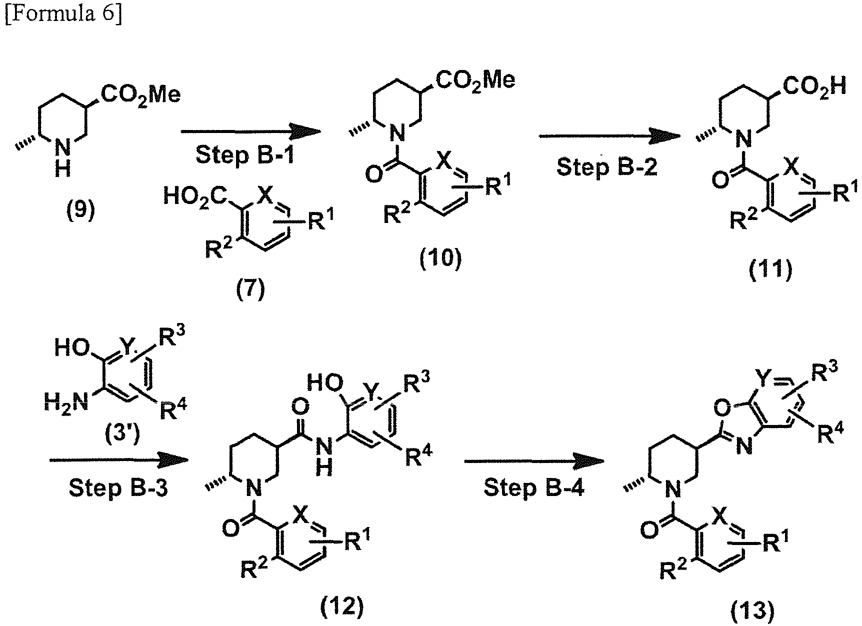
wherein X, Y, R¹, R², and R³ are as defined above.
Step B-1: The compound (10) can be obtained through the condensation reaction of a compound (9) and a compound (7). The condensation reaction in Step B-1 can be performed according to the same reaction conditions as in Step A-2.
Step B-2: The compound (11) can be obtained through the hydrolysis reaction of ester in the compound (10). The reaction in Step B-2 can be performed according to the same reaction conditions as in Step A-1. Alternatively, the compound (11) may be obtained through acid hydrolysis using an aqueous solution of a mineral acid such as hydrochloric acid or sulfuric acid. A mixed solvent of methanol, ethanol, 1,4-dioxane and the like can be used as a solvent. This reaction can be performed under conditions of 0°C to reflux temperature.
Step B-3: The compound (12) can be obtained through the condensation reaction of the compound (11) and a compound (3'). The condensation reaction in Step B-3 can be performed according to the same reaction conditions as in Step A-2.
Step B-4: The compound (13) can be obtained through the ring-closing reaction of the compound (12). The reaction in Step B-4 can be performed according to the same reaction conditions as in Step A-3. 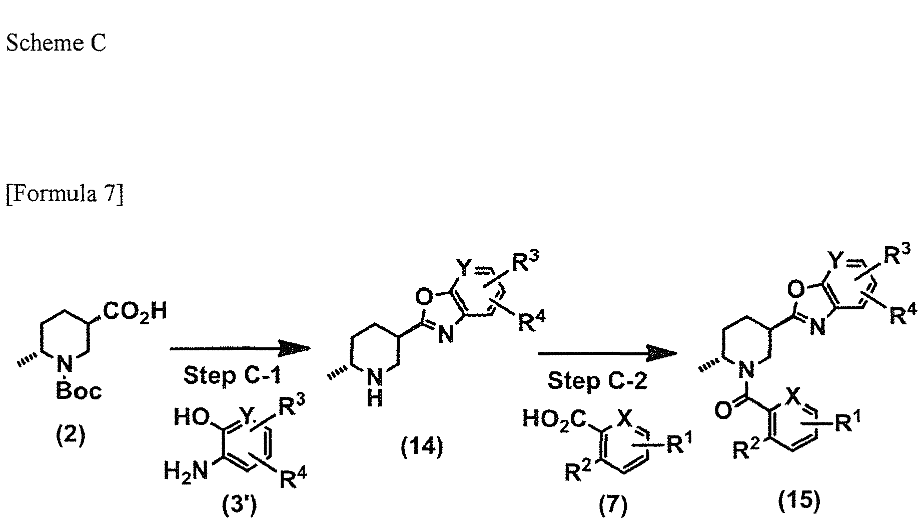
wherein X, Y, R¹, R², and R³ are as defined above.
Step C-1: The compound (14) can be obtained through the cyclization reaction of the compound (2) and the compound (3'). The reaction in Step C-1 can be performed under conditions of 80 to 120°C using an Eaton reagent as an acid catalyst.
Step C-2: The compound (15) can be obtained through the condensation reaction of the compound (14) and the compound (7). The condensation reaction in Step C-2 can be performed according to the same reaction conditions as in Step A-2.

### Examples

Hereinafter, the present invention will be described in more detail with reference to Reference Examples, Examples, and Test Examples. However, the present invention is not limited by these examples, and various changes or modifications can be made without departing from the scope of the present invention. In Reference Examples and Examples below, "KP-Sil" used in purification using column chromatography means SNAP Cartridge KP-Sil manufactured by Biotage AB; "HP-Sil" used therein means SNAP Cartridge HP-Sil manufactured by Biotage AB; and "KP-NH" used therein means SNAP Cartridge KP-NH manufactured by Biotage AB.

In Reference Examples and Examples below, purification by preparative high-performance liquid chromatography (HPLC) was performed under the following conditions, provided that in the case of compounds having basic functional groups, neutralization operation or the like for obtaining free forms may be performed when trifluoroacetic acid is used in this operation:
Apparatus: preparative HPLC system manufactured by Gilson, Inc.
Column: Capcell Pak C18 MGII 5 µm 20 x 150 mm manufactured by Shiseido Co., Ltd.
Solvent: Solution A; water containing 0.1% trifluoroacetic acid, Solution B; acetonitrile containing 0.1% trifluoroacetic acid
Gradient: 0 min. (Solution A/Solution B = 90/10), 22 min. (Solution A/Solution B = 20/80), 25 min. (Solution A/Solution B = 10/90)
Flow rate: 20 mL/min, Detection method: UV 254 nm

In Reference Examples and Examples below, high-performance liquid chromatography mass spectrometry (LCMS) was carried out under any of the following four types of conditions:

### • Condition 1

Measurement apparatus: Waters-MicroMass Platform LC and Agilent 1100 manufactured by Agilent Technologies, Inc.
Column: SunFire C18 2.5 µm 4.6 x 50 mm manufactured by Waters Corp.
Solvent: water containing 0.1% trifluoroacetic acid, Solution B; acetonitrile containing 0.1% trifluoroacetic acid
Gradient: 0 min. (Solution A/Solution B = 90/10), 0.5 min. (Solution A/Solution B = 90/10), 5.5 min. (Solution A/Solution B = 20/80), 6.0 min. (Solution A/Solution B = 1/99), 6.3 min.
(Solution A/Solution B = 1/99)
Flow rate: 1 mL/min, Detection method: 254 nm
Ionization method: electron spray ionization (ESI)

### • Condition 2

Measurement apparatus: LCMS-2010EV manufactured by Shimadzu Corp.
Column: Shimpack XR-ODS 2.2 µm 2.0 x 30 mm manufactured by Shimadzu Corp.
Solvent: Solution A; water containing 0.1% formic acid, Solution B; acetonitrile containing 0.1% formic acid
Gradient: 0 min. (Solution A/Solution B = 90/10), 1 min. (Solution A/Solution B = 60/40), 2 min.
(Solution A/Solution B = 0/100), 2.5 min. (Solution A/Solution B = 0/100)
Flow rate: 0.6 mL/min, Detection method: 254 nm
Ionization method: electron spray ionization (ESI) and atmospheric pressure chemical ionization

### (APCI)

### • Condition 3

Measurement apparatus: LCMS-2010EV manufactured by Shimadzu Corp.
Column: Shimpack XR-ODS 2.2 µm 2.0 x 30 mm manufactured by Shimadzu Corp.
Solvent: Solution A; water containing 0.1% formic acid, Solution B; acetonitrile containing 0.1% formic acid
Gradient: 0 min. (Solution A/Solution B = 90/10), 3 min. (Solution A/Solution B = 60/40), 5.5 min. (Solution A/Solution B = 0/100), 6.5 min. (Solution A/Solution B = 0/100)
Flow rate: 0.6 mL/min, Detection method: 254 nm
Ionization method: electron spray ionization (ESI) and atmospheric pressure chemical ionization

### (APCI)

### • Condition 4

Measurement apparatus: Agilent 2900 and Agilent 6150 manufactured by Agilent Technologies, Inc.
Column: Acquity CSH C18 1.7 µm 2.1 x 50 mm manufactured by Waters Corp.
Solvent: Solution A; water containing 0.1% formic acid, Solution B; acetonitrile containing 0.1% formic acid
Gradient: 0 min. (Solution A/Solution B = 80/20), 1.2 to 1.4 min. (Solution A/Solution B = 1/99) Flow rate: 0.8 mL/min, Detection method: UV 254 nm

### Ionization method: electron spray ionization (ESI)

In Examples below, racemic resolution was carried out under any of the following three types of conditions:

### • Condition 1

Column: CHIRALPAK AD (Daicel Corp.), 20 mm x 250 mm
Mobile phase: hexane/ethanol = 40/60 (v/v)
Flow rate: 5.0 mL/min

### • Condition 2

Column: CHIRALPAK AD (Daicel Corp.), 20 mm x 250 mm
Mobile phase: hexane/ethanol = 80/20 (v/v) to 0/100 (v/v)
Flow rate: 5.0 mL/min

### • Condition 3

Column: CHIRALPAK IC (Daicel Corp.), 10 cm x 25 cm
Mobile phase: hexane/2-propanol = 90/10 (v/v)
Flow rate: 142 mL/min.

In Examples below, chiral analysis was carried out under the following conditions:

### • Condition A

Column: CHIRALPAK AD-H (Daicel Corp.), 4.6 mm x 250 mm

### Flow rate: 1.0 mL/min

### Mobile phase: hexane/ethanol = 50/50

In Reference Examples and Examples below, mass spectrometry (MS) was carried out under the following conditions:
MS measurement apparatus: LCMS-2010EV manufactured by Shimadzu Corp. or Waters-MicroMass Platform LC

In Reference Examples and Examples below, compounds were designated using ACD/Name (ACD/Labs 12.01, Advanced Chemistry Development Inc.).

Terms and reagents indicated in Reference Examples and Examples are defined as follows:
Na₂SO₄ (anhydrous sodium sulfate), MgSO₄ (anhydrous magnesium sulfate), Na₂CO₃ (sodium carbonate), Cs₂CO₃ (cesium carbonate), NaHCO₃ (sodium bicarbonate), NaOH (sodium hydroxide), LiOH·H₂O (lithium hydroxide monohydrate), MeOH (methanol), EtOH (ethanol),
Et₂O (diethyl ether), THF (tetrahydrofuran), DMF (N,N-dimethylformamide), MeCN (acetonitrile), EtOAc (ethyl acetate), CHCl₃ (chloroform), HOBt·H₂O (1-hydroxybenzotriazole monohydrate), EDC·HCl [1-(3-dimethylaminopropyl)-3-ethylcarbodiimide monohydrochloride],
HATU [O-(7-azabenzotriazol-1-yl)-N,N,N',N'-tetramethyluronium hexafluorophosphate], DMT-MM (4-(4,6-dimethoxy-1,3,5-triazin-2-yl)-4-methylmorpholinium chloride), Pd(PPh₃)₄ [tetrakis(triphenylphosphine)palladium(0)], brine (saturated saline), Boc (tert-butoxycarbonyl),
THP (tetrahydropyranyl), DIPEA (N,N-diisopropylethylamine), TEA (triethylamine), MeI (methyl iodide), EtI (ethyl iodide), TBAF (tetrabutylammonium fluoride), TBTU (O-(benzotriazol-1-yl)-N,N,N',N'-tetramethyluronium tetrafluoroborate), MsCl (methane sulfonyl chloride), NaBH₄ (sodium borohydride), NaH (sodium hydride), HCl (hydrogen chloride), H₂O (water), PPA (polyphosphoric acid), and PTLC (preparative thin-layer chromatography).

### Reference Example 1 (3RS,6RS)-1-(Tert-butoxycarbonyl)-6-methylpiperidine-3-carboxylic acid

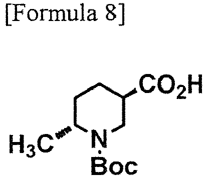

LiOH·H₂O (2.74 g, 65.3 mmol) was added to a THF (497 mL)/H₂O (124 mL) mixed solution of methyl (3RS,6RS)-1-(tert-butoxycarbonyl)-6-methylpiperidine-3-carboxylate (16.0 g, 62.2 mmol), and the mixture was stirred overnight at room temperature. H₂O was added thereto, followed by extraction with Et₂O. Then, 2 mol/L hydrochloric acid was added to the aqueous layer, followed by extraction using EtOAc. The obtained organic layer was dried over MgSO₄, and the desiccant was then filtered off. The solvent was distilled off under reduced pressure to obtain the title compound (12.7 g) (colorless solid).
MS (ESI neg.) m/z: 242 [M-H]-

### Reference Example 2 Tert-butyl (2RS,5RS)-5-[(5-chloro-2-hydroxyphenyl)carbamoyl]-2-methylpiperidine-1-carboxylate

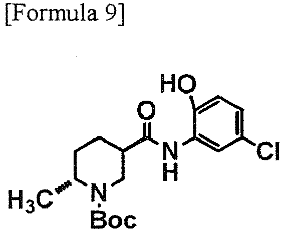

DMT-MM (521 mg, 1.77 mmol) was added to an EtOH solution (18 mL) of 4-chloro-2-aminophenol (254 mg, 1.77 mmol) and (3RS,6RS)-1-(tert-butoxycarbonyl)-6-methylpiperidine-3-carboxylic acid (331 mg, 1.36 mmol) obtained in Reference Example 1, and the mixture was stirred at room temperature for 3 days. The reaction solution was concentrated, and H₂O was then added to the residue, followed by extraction using EtOAc. The organic layer was dried over MgSO₄, and the desiccant was then filtered off. The solvent was distilled off under reduced pressure. The obtained residue was purified by column chromatography (HP-Sil 25 g, hexane/EtOAc) to obtain the title compound (331 mg) (brown amorphous form).
MS (ESI pos.) m/z : 369 [M+H]+

### Reference Example 3 Tert-butyl (2RS,5RS)-5-(5-chloro-1,3-benzoxazol-2-yl)-2-methylpiperidine-1-carboxylate

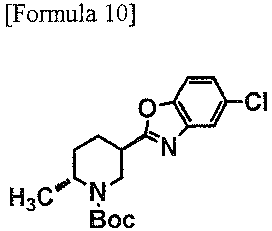

Pyridine (1.39 mL, 17.2 mmol) and thionyl chloride (0.626 mL, 8.6 mmol) were added to a toluene solution (8.6 mL) oftert-butyl (2RS,5RS)-5-[(5-chloro-2-hydroxyphenyl)carbamoyl]-2-methylpiperidine-1-carboxylate (317 mg, 0.86 mmol), and the mixture was stirred at 100°C for 2 hours. Pyridine (1.39 mL, 17.2 mmol) and thionyl chloride (0.626 mL, 8.6 mmol) were further added to the reaction solution, and the mixture was stirred at 100°C for 2 hours. After standing to cool to room temperature, H₂O was added to the reaction mixture, followed by extraction using EtOAc. The obtained organic layer was dried over MgSO₄, and the desiccant was then filtered off. The solvent was distilled off under reduced pressure. The obtained residue was purified by column chromatography (HP-Sil 25 g, hexane/EtOAc) to obtain the title compound (158 mg) (colorless amorphous form).
MS (ESI pos.) m/z : 351 [M+H]+

### Reference Example 4 5-Chloro-2-[(3RS,6RS)-6-methylpiperidin-3-yl]-1,3-benzoxazole

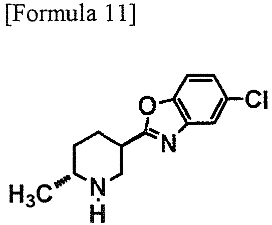

A 4 mol/L HCl-EtOAc solution (2.3 mL, 9.16 mmol) was added to an EtOAc solution (4.6 mL) of tert-butyl (2RS,5RS)-5-(5-chloro-1,3-benzoxazol-2-yl)-2-methylpiperidine-1-carboxylate (160.7 mg, 0.46 mmol), and the mixture was stirred overnight at room temperature. The reaction mixture was concentrated under reduced pressure, and a 2 mol/L aqueous NaOH solution was added to the residue, followed by extraction using EtOAc. The obtained organic layer was dried over MgSO₄, and the desiccant was then filtered off. The solvent was distilled off under reduced pressure. The obtained residue was purified by column chromatography (HP-Sil 10 g, hexane/EtOAc) to obtain the title compound (108 mg) (colorless amorphous form). MS (ESI pos.) m/z : 251 [M+H]+

### Reference Example 5 Tert-butyl (2RS,5RS)-5-[(5-fluoro-2-hydroxyphenyl)carbamoyl]-2-methylpiperidine-1-carboxylate

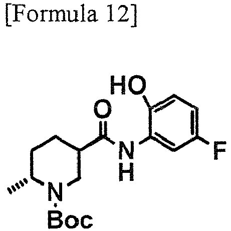

The title compound (799 mg) was obtained (brown amorphous form) by the same approach as in Reference Example 2 using (3RS,6RS)-1-(tert-butoxycarbonyl)-6-methylpiperidine-3-carboxylic acid (810 mg, 3.33 mmol) obtained in Reference Example 1 and 4-fluoro-2-aminophenol (1.1 g, 8.66 mmol) as starting materials.
MS (ESI pos.) m/z: 431 [M+H]+

### Reference Example 6 Tert-butyl (2RS,5RS)-5-(5-fluoro-1,3-benzoxazol-2-yl)-2-methylpiperidine-1-carboxylate

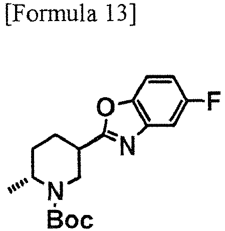

The title compound (329 mg) was obtained (colorless amorphous form) by the same approach as in Reference Example 2 using tert-butyl (2RS,5RS)-5-[(5-fluoro-2-hydroxyphenyl)carbamoyl]-2-methylpiperidine-1-carboxylate (763 mg, 2.07 mmol) obtained in Reference Example 5, pyridine (6.68 mL, 41.4 mmol), and thionyl chloride (3.02 mL, 41.4 mmol. MS (ESI pos.) m/z: 335 [M+H]+

### Reference Example 7 5-Fluoro-2-[(3RS,6RS)-6-methylpiperidin-3-yl]-1,3-benzoxazole

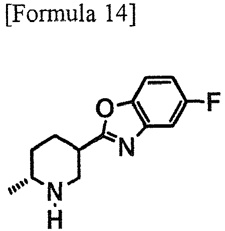

The title compound (190 mg) was obtained (colorless amorphous form) by the same approach as in Reference Example 4 using tert-butyl (2RS,5RS)-5-(5-fluoro-1,3-benzoxazol-2-yl)-2-methylpiperidine-1-carboxylate (329 mg, 0.94 mmol) obtained in Reference Example 6 and a 4 mol/L HCl-EtOAc solution (4.7 mL, 18.8 mmol).
MS (ESI pos.) m/z: 235 [M+H]+

### Reference Example 8 Methyl (3RS,6RS)-6-methyl-1-[5-methyl-2-(2H-1,2,3-triazol-2-yl)benzoyl]piperidine-3-carboxylate

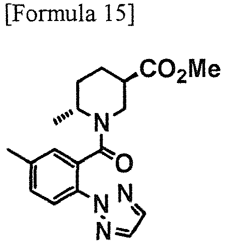

DIPEA (20.6 mL, 118 mmol) and HATU (10.6 g, 32.5 mmol) were added to a DMF solution (295 mL) of methyl (3RS,6RS)-6-methylpiperidine-3-carboxylate (4.64 g, 29.5 mmol) and 5-methyl-2-(2H-1,2,3-triazol-2-yl)benzoic acid (6.00 g, 29.5 mmol), and the mixture was stirred overnight at room temperature. Water was added to the reaction mixture, followed by extraction using EtOAc. The solvent in the organic layer was distilled off under reduced pressure. The obtained residue was purified by column chromatography (HP-Sil 100 g, Hexane/EtOAc) to obtain the title compound (2.83 g) (colorless oil).
MS (ESI pos.) m/z : 297 [M+H]+

### Reference Example 9 (3RS,6RS)-6-Methyl-1-[5-methyl-2-(2H-1,2,3-triazol-2-yl)benzoyl]piperidine-3-carboxylic acid

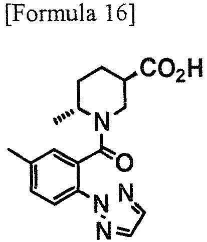

2 mol/L hydrochloric acid (57.0 mL) was added to a 1,4-dioxane (497 mL) solution of methyl (3RS,6RS)-6-methyl-1-[5-methyl-2-(2H-1,2,3-triazol-2-yl)benzoyl]piperidine-3-carboxylate (329 mg, 0.94 mmol) obtained in Reference Example 8, and the mixture was stirred at 80°C for 6 hours. The reaction mixture was concentrated under reduced pressure. The obtained residue was purified by column chromatography (HP-Sil 50 g, hexane/EtOAc) to obtain the title compound (1.41 g) (colorless amorphous form).
MS (ESI pos.) m/z: 329 [M+H]+

### Reference Example 10 (3RS,6RS)-N-(5-Chloro-2-hydroxyphenyl)-6-methyl-1-[5-methyl-2-(2H-1,2,3-triazol-2-yl)benzoyl]piperidine-3-carboxamide

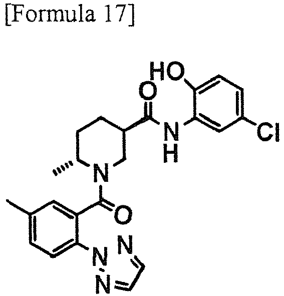

The title compound (118 mg) was obtained (brown oil) by the same approach as in Reference Example 8 using (3RS,6RS)-6-methyl-1-[5-methyl-2-(2H-1,2,3-triazol-2-yl)benzoyl]piperidine-3-carboxylic acid (300 mg, 0.91 mmol) obtained in Reference Example 9 and 4-chloro-2-aminophenol (157 mg, 1.10 mmol) as starting materials.
MS (ESI pos.) m/z : 454 [M+H]+

### Reference Example 11 (3RS,6RS)-N-(5-Fluoro-2-hydroxyphenyl)-6-methyl-1-[5-methyl-2-(2H-1,2,3-triazol-2-yl)benzoyl]piperidine-3-carboxamide

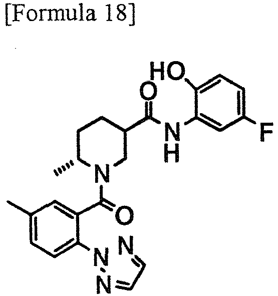

HOBt·H₂O (109 mg, 0.80 mmol) and EDC·HCl (140 mg, 0.73 mmol) were added to a DMF solution (6 mL) of 4-fluoro-2-aminophenol (85.2 mg, 0.67 mmol) and (3RS,6RS)-6-methyl-1-[5-methyl-2-(2H-1,2,3-triazol-2-yl)benzoyl]piperidine-3-carboxylic acid (200 mg, 0.61 mmol) obtained in Reference Example 9, and the mixture was stirred overnight at room temperature. An aqueous NaHCO₃ solution was added to the reaction solution, followed by extraction using EtOAc. The organic layer was dried over MgSO₄, and the desiccant was then filtered off. The solvent was distilled off under reduced pressure. The obtained residue was purified (67 mg) by column chromatography (HP-Sil 25 g, hexane/EtOAc). DIPEA (0.6 mL) was added to a DMF solution (0.6 mL) of a portion (26 mg) of the obtained compound, and the mixture was stirred overnight at 100°C. After standing to cool to room temperature, H₂O was added thereto, followed by extraction using EtOAc. The obtained organic layer was dried over MgSO₄, and the desiccant was then filtered off. The solvent was distilled off under reduced pressure. The obtained residue was purified by column chromatography (HP-Sil 10 g, hexane/EtOAc and then KP-NH 11 g, CHCl₃/MeOH) to obtain the title compound (18 mg) (colorless amorphous form).
MS (ESI pos.) m/z : 438 [M+H]+

### Reference Example 12 5-Fluoro-2-[(3RS,6RS)-6-methylpiperidin-3-yl]-1,3-benzoxazole

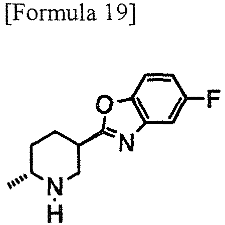

A mixture of 4-fluoro-2-aminophenol (575 mg, 4.52 mmol), (3RS,6RS)-1-(tert-butoxycarbonyl)-6-methylpiperidine-3-carboxylic acid (1.0 g, 4.11 mmol) obtained in Reference Example 1, and PPA (2.5 g) was stirred at 180°C for 30 minutes. After standing to cool to room temperature, a saturated aqueous solution of NaHCO₃ was added to the reaction solution, followed by extraction using EtOAc. The organic layer was dried over MgSO₄, and the desiccant was then filtered off. The solvent was distilled off under reduced pressure. The obtained residue was purified (672 mg) by column chromatography (HP-Sil 50 g, hexane/EtOAc). TEA (0.70 mL, 5.02 mmol) and Boc₂O (1.1 g, 5.02 mmol) were added to a CHCl₃ solution (13 mL) of a portion (608 mg) of the obtained compound, and the mixture was stirred at 50°C for 1.5 hours. After standing to cool to room temperature, 2 mol/L hydrochloric acid was added thereto, followed by extraction using EtOAc. The obtained organic layer was dried over MgSO₄, and the desiccant was then filtered off. The solvent was distilled off under reduced pressure. The obtained residue was purified (333 mg) by column chromatography (HP-Sil 50 g, hexane/EtOAc). A 4 mol/L HCl-EtOAc solution (5.0 mL, 19.9 mmol) was added to an EtOAc solution (5.0 mL) of the obtained compound (333 mg, 1.00 mmol), and the mixture was stirred at room temperature for 2 hours. The reaction mixture was concentrated under reduced pressure, and a 2 mol/L aqueous NaOH solution was added to the residue, followed by extraction using EtOAc. The obtained organic layer was dried over MgSO₄, and the desiccant was then filtered off The solvent was distilled off under reduced pressure. The obtained residue was purified by column chromatography (KP-NH 10 g, hexane/EtOAc) to obtain the title compound (196 mg) (colorless amorphous form).
MS (ESI pos.) m/z : 235 [M+H]+

### Reference Example 13 6-Fluoro-2-[(3RS,6RS)-6-methylpiperidin-3-yl]-1,3-benzoxazole hydrochloride

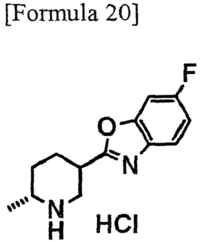

A mixture of 4-fluoro-2-aminophenol (575 mg, 4.52 mmol), (3RS,6RS)-1-(tert-butoxycarbonyl)-6-methylpiperidine-3-carboxylic acid (1.0 g, 4.11 mmol) obtained in Reference Example 1, and PPA (2.5 g) was stirred at 180°C for 30 minutes. After standing to cool to room temperature, an aqueous NaHCO₃ solution was added to the reaction solution, followed by extraction using EtOAc. The organic layer was dried over MgSO₄, and the desiccant was then filtered off The solvent was distilled off under reduced pressure. The obtained residue was purified (560 mg) by column chromatography (HP-Sil 50 g, hexane/EtOAc). TEA (0.37 mL, 2.63 mmol) and Boc₂O (0.57 g, 2.63 mmol) were added to a THF solution (20 mL) of the obtained compound in the whole amount (560 mg), and the mixture was stirred at room temperature for 2 hours and at 50°C for 1 hour. After standing to cool to room temperature, 2 mol/L hydrochloric acid was added thereto, followed by extraction using EtOAc. The obtained organic layer was dried over MgSO₄, and the desiccant was then filtered off. The solvent was distilled off under reduced pressure. The obtained residue was purified (464 mg) by column chromatography (HP-Sil 50 g, hexane/EtOAc). A 4 mol/L, HCl-EtOAc solution (7.0 mL, 27.8 mmol) was added to an EtOAc solution (5.6 mL) of the obtained compound in the whole amount (464 mg, 1.39 mmol), and the mixture was stirred at room temperature for 3 hours. The formed solid was collected by filtration to obtain the title compound (343 mg) (colorless solid).
MS (ESI pos.) m/z : 235 [M+H]⁺

### Reference Example 14 Methyl (3R,6R)-1-(tert-butoxycarbonyl)-6-methylpiperidine-3-carboxylate

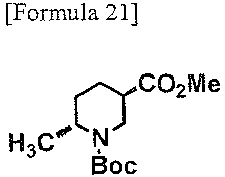

A racemic mixture of methyl (3RS,6RS)-1-(tert-butoxycarbonyl)-6-methylpiperidine-3-carboxylate (80.0 g, 329 mmol) was resolved under the above racemic resolution conditions (Condition 3) to separate two peaks (retention time: 28.5 min and 35.8 min). Of them, the title compound (34.9 g) was obtained (colorless oil) as a compound having a longer relative retention time (retention time: 35.8 min). The obtained title compound was converted to [(2R,5R)-5-(5-chloro-1,3-benzoxazol-2-yl)-2-methylpiperidin-1-yl][6-methyl-3-(2H-1,2,3-triazol-2-yl)pyridin-2-yl]methanone using the same approach as in Reference Examples 1, 16, 24, and 32 and Example 1. Its absolute stereostructure was determined by X-ray crystal structural analysis. MS (ESI pos.) m/z : 244 [M+H]+

### Reference Example 15 (3R,6R)-1-(Tert-butoxycarbonyl)-6-methylpiperidine-3-carboxylic acid

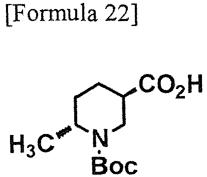

LiOH·H₂O (1.01 g, 24.1 mmol) was added to a THF (183 mL)/H₂O (46 mL) mixed solution of methyl (3R,6R)-1-(tert-butoxycarbonyl)-6-methylpiperidine-3-carboxylate (5.9 g, 22.9 mmol), and the mixture was stirred overnight at room temperature. H₂O was added thereto, followed by extraction with Et₂O. Then, 2 mol/L hydrochloric acid was added to the aqueous layer, followed by extraction using EtOAc. The obtained organic layer was dried over MgSO₄, and the desiccant was then filtered off. The solvent was distilled off under reduced pressure to obtain the title compound (5.41 g) (colorless solid).
MS (ESI neg.) m/z: 242 [M-H]-

### Reference Example 16 Tert-butyl (2R,5R)-5-{[2-hydroxy-5-(trifluoromethyl)phenyl]carbamoyl}-2-methylpiperidine-1-carboxylate

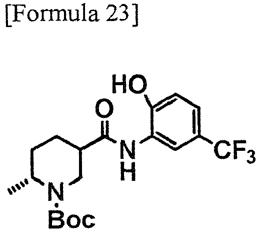

HOBt·H₂O (225 mg, 1.48 mmol) and EDC·HCl (283 mg, 1.48 mmol) were added to a DMF solution (12 mL) of 5-trifluoromethyl-2-triethylsilyloxyaniline (395 mg, 1.36 mmol) and (3R,6R)-1-(tert-butoxycarbonyl)-6-methylpiperidine-3-carboxylic acid (300 mg, 1.23 mmol) obtained in Reference Example 15, and the mixture was stirred overnight at room temperature. H₂O was added to the reaction solution, followed by extraction using Et₂O. The organic layer was dried over MgSO₄, and the desiccant was then filtered off. The solvent was distilled off under reduced pressure. The obtained residue was dissolved in THF (30 mL). TBAF (1.0 mol/L THF solution, 2.5 mL, 2.5 mmol) was added to the solution at room temperature, and the mixture was stirred for 2 hours. H₂O was added thereto, followed by extraction using EtOAc. The solvent was distilled off under reduced pressure. The obtained residue was purified by column chromatography (HP-Sil 100 g, hexane/EtOAc) to obtain the title compound (562 mg) (pale yellow oil).
MS (ESI pos.) m/z : 425 [M+Na]⁺

The following compounds of Reference Examples 17 to 23 were obtained by the same approach as in Reference Example 16. The structural formulas, names, and MS data of the obtained compounds are shown in Table 1.

**[Table 1]**

| Reference Example No | Structural formula | Compound name | MS (ESI) m/z |
|---|---|---|---|
| 17 | 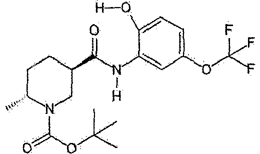 | Tert-butyl (2R,5R)-5-{[2-hydroxy-5-(trifluoromethoxy)phenyl]carbamoyl]-2-methylpiperidine-1-carboxylate | 417(M-H)- |
| 18 | 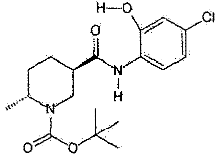 | Tert-butyl(2R,5R)-5-[(4-chloro-2-hydroxyphenyl)carbamoyl]-2-methylpiperidine-1-carboxylate | 391 (M+Na)+ |
| 19 | 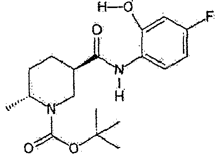 | Tert-butyl (2R.5R)-5-[(4-fluoro-2-hydroxyphenyl)carbamoyl]-2-methylpiperidine-1-carboxylate | 375(M+Na)+ |
| 20 | 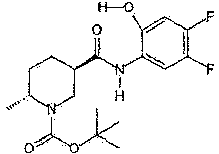 | Tert-butyl (2,5R)-5-[(4,5-difluoro-2-hydroxyphenyl)carbamoyl]-2-methylpiperidine-1-carboxylate | 393(M+Na)+ |
| 21 | 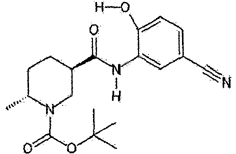 | Tert-butyl (2R,5R)-5-[(5-cyano-2-hydroxyphenyl)carbamoyl]-2-methylpiperidine-1-carboxylate | 382(M+Na)+ |
| 22 | 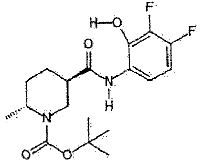 | Tert-butyl (2R,5R)-5-[(3,4-difluoro-2-hydroxyphenyl)carbamoyl]-2-methylpiperidine-1-carboxylate | 393(M+Na)+ |
| 23 | 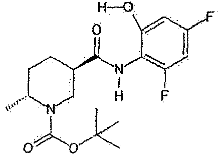 | Tert-butyl(2R,5R)-5-[(2,4-difluoro-6-hydroxyphenyl)carbamoyl]-2-methylpiperidine-1-carboxylate | 393(M+Na)+ |

### Reference Example 24 Tert-butyl (2R,5R)-2-methyl-5-[5-(trifluoromethyl)-1,3-benzoxazol-2-yl]piperidine-1-carboxylate

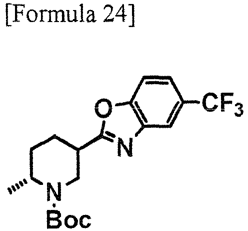

DEAD (2.2 mol/L, 1.23 mL, 2.71 mmol) was added to a THF solution (12 mL) of tert-butyl (2R,5R)-5-{[2-hydroxy-5-(trifluoromethyl)phenyl]carbamoyl}-2-methylpiperidine-1-carboxylate (494 mg, 1.23 mmol) and PPh₃ (710 mg, 2.71 mmol) under ice cooling, and the mixture was stirred overnight at room temperature. After concentration under reduced pressure, the residue was purified by HPLC to obtain the title compound (244 mg) (pale yellow oil).
MS (ESI pos.) m/z : 385 [M+H]+

The following compounds of Reference Examples 25 to 31 were obtained by the same approach as in Reference Example 24. The structural formulas, names, and MS data of the obtained compounds are shown in Table 2.

**[Table 2]**

| Reference Example No | Structural formula | Compound name | MS (ESI pos.) m/z |
|---|---|---|---|
| 25 | 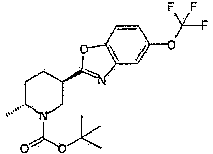 | Tert-butyl (2R,5R)-2-methyl-5-[5-(trifluoromethoxy)-1,3-benzoxazol-2-yl] piperidine-1-carboxylate | 401(M+H)+ |
| 26 | 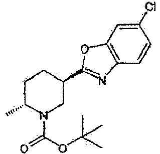 | Tert-butyl (2R,5R)-5-(6-chloro-1,3-benzoxazol-2-yl)-2-methylpiperidine-1-carboxylate | 351(M+H)+ |
| 27 | 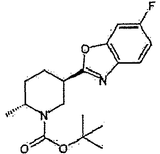 | Tert-butyl (2R,5R)-5-(6-fluoro-1,3-benzoxazol-2-yl)-2-methylpiperidine-1-carboxylate | 335(M+H)+ |
| 28 | 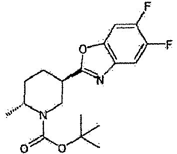 | Tert-butyl (2R,5R)-5-(5,6-difluoro-1,3-benzoxazol-2-yl)-2-methylpiperidine-1-carboxylate | 353(M+H)+ |
| 29 | 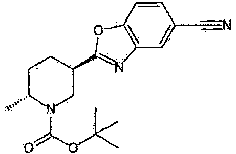 | Tert-butyl (2R,5R)-5-(5-cyano-1,3-benzoxazol-2-yl)-2-methylpiperidine-1-carboxylate | 364(M+Na)+ |
| 30 | 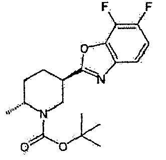 | Tert-butyl (2R,5R)-5-(6,7-difluoro-1,3-benzoxazol-2-yl)-2-methylpiperidine-1-carboxylate | 353(M+H)+ |
| 31 | 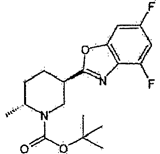 | Tert-butyl (2R,5R)-5-4,6-difluoro-1,3-benzoxazol-2-yl)-2-methylpiperidine-1-carboxylate | 375(M+Na)+ |

### Reference Example 32 2-[(3R,6R)-6-Methylpiperidin-3-yl]-5-(trifluoromethyl)-1,3-benzoxazole hydrochloride

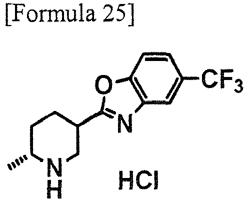

A 4 mol/L HCl-EtOAc solution (6.3 mL) was added to an EtOAc solution (6.3 mL) of tert-butyl (2R,5R)-2-methyl-5-[5-(trifluoromethyl)-1,3-benzoxazol-2-yl]piperidine-1-carboxylate (244 mg, 0.635 mmol), and the mixture was stirred at room temperature for 30 minutes. The reaction mixture was concentrated under reduced pressure to obtain the title compound (195 mg) (colorless solid).
MS (ESI pos.) m/z: 285 [M+H]+

The following compounds of Reference Examples 33 to 39 were obtained by the same approach as in Reference Example 32. The structural formulas, names, and MS data of the obtained compounds are shown in Table 3.

**[Table 3]**

| Reference Example No | Structural formula | Compound name | MS (ESI pos.) m/z |
|---|---|---|---|
| 33 | 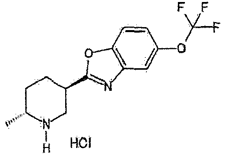 | 2-[(3R,6R)-6-Methylpiperidin-3-yl]-5-(trifluoromethoxy)-1,3-benzoxazole hydrochloride | - |
| 34 | 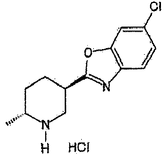 | 6-Chloro-2-[(3R,6R)-6-methylpiperidin-3-yl]-1,3-benzoxazole hydrochloride | 251(M+H)+ |
| 35 | 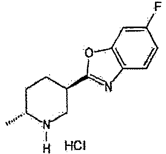 | 6-Fluoro-2-[(3R,6R)-6-methylpiperidin-3-yl]-1,3-benzoxazole hydrochloride | 235(Nt+H)+ |
| 36 | 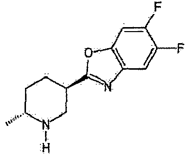 | 5,6-Difluoro-2-[(3R,6R)-6-methylpiperidin -3-yl]-1,3-benzoxazole | 253(M+H)+ |
| 37 | 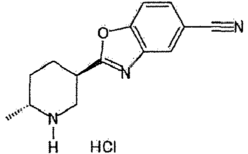 | 2-[(3R,6R)-6-Methylpiperidin-3-yl]-1,3-benzoxazole-5-carbonitrile hydrochloride | 242(M+H)+ |
| 38 | 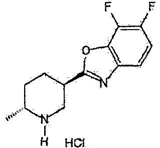 | 6,7-Difluoro-2-[(3R,6R)-6-methylpiperidin -3-yl]-1,3-benzoxazole hydrochloride | 253(M+H)+ |
| 39 | 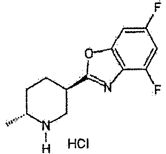 | 4,6-Difluoro-2-[(3R,6R)-6-methylpiperidin -3-yl]-1,3-benzoxazole hydrochloride | 253(M+H)+ |

### Reference Example 40 5,7-Difluoro-2-[(3R,6R)-6-methylpiperidin-3-yl]-1,3-benzoxazole

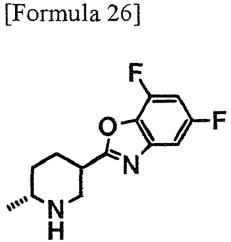

A mixture of 4,6-difluoro-2-aminophenol (66 mg, 0.411 mmol), (3R,6R)-1-(tert-butoxycarbonyl)-6-methylpiperidine-3-carboxylic acid (100 mg, 0.411 mmol) obtained in Reference Example 1, and an Eaton reagent (0.667 mL) was stirred at 80°C for 60 minutes under microwave irradiation. After standing to cool to room temperature, an aqueous NaHCO₃ solution was added to the reaction solution, followed by extraction using CHCl₃. The obtained residue was purified by PTLC (NH silica 0.95 mm x 1, hexane/EtOAc) to obtain the title compound (56 mg) (colorless solid).

### MS (ESI pos.) m/z : 253 [M+H]+

The following compounds of Reference Examples 41 to 43 were obtained by the same approach as in Reference Example 40. The structural formulas, names, and MS data of the obtained compounds are shown in Table 4.

**[Table 4]**

| Reference Example No | Structural formula | Compound name | MS (ESI pos.) m/z |
|---|---|---|---|
| 41 | 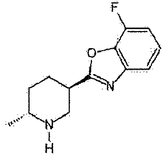 | 7-Fluoro-2-[(3R,6R)-6-methylpiperidin-3-yl]-1,3-benzoxazole | 235(M+H)+ |
| 42 | 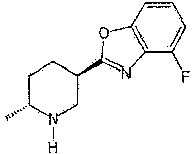 | 4-Fluoro-2-[(3R,6R)-6-methylpiperidin-3-yl]-1,3-benzoxazole | 235(M+H)+ |
| 43 | 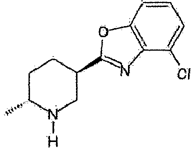 | 4-Chloro-2-[(3R,6R)-6-methylpiperidin-3-yl]-1,3-benzoxazole | 251(M+H)+ |

### Reference Example 44 (3R,6R)-N-(5-Chloro-2-hydroxypyridin-3-yl)-6-methyl-1-[5-methyl-2-(2H-1,2,3-triazol-2-yl)benzoyl]piperidine-3-carboxamide

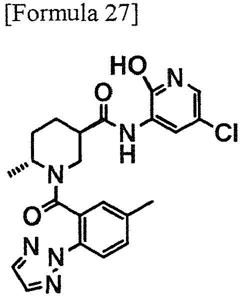

The title compound (50 mg) was obtained (brown oil) by the same approach as in Reference Examples 8 to 10 using methyl (3R,6R)-6-methylpiperidine-3-carboxylate hydrochloride (165 mg, 0.78 mmol) converted from methyl (3R,6R)-1-(tert-butoxycarbonyl)-6-methylpiperidine-3-carboxylate and 3-amino-5-chloro-2-hydroxypyridine hydrochloride (143 mg, 0.79 mmol) as starting materials.

### MS (ESI pos.) m/z: 455 [M+H]+

### Reference Example 45 Methyl (3R,6R)-1-[4-fluoro-2-(2H-1,2,3-triazol-2-yl)benzoyl]-6-methylpiperidine-3-carboxylate

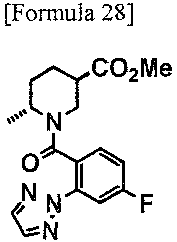

DIPEA (0.182 mL, 1.04 mmol) and HATU (102.0 mg, 0.313 mmol) were added to a DMF (2.61 mL) solution of methyl (3R,6R)-6-methylpiperidine-3-carboxylate (41.0 mg, 0.261 mmol) converted from methyl (3R,6R)-1-(tert-butoxycarbonyl)-6-methylpiperidine-3-carboxylate and 4-fluoro-2-(2H-1,2,3-triazol-2-yl)benzoic acid (54.0 mg, 0.261 mmol), and the mixture was stirred overnight at room temperature. An aqueous NaHCO₃ solution was added to the reaction mixture, followed by extraction using CHCl₃. The solvent in the organic layer was distilled off under reduced pressure. The obtained residue was purified by column chromatography (HP-Sil, Hexane/EtOAc) to obtain the title compound (73.0 mg) (colorless amorphous form).
MS (ESI pos.) m/z : 347 [M+H]+

### Reference Example 46 (3R,6R)-1-[4-Fluoro-2-(2H-1,2,3-triazol-2-yl)benzoyl]-6-methylpiperidine-3-carboxylic acid

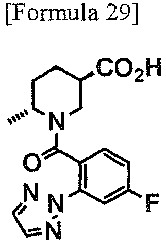

2 mol/L hydrochloric acid (2.1 mL) was added to a 1,4-dioxane (2.1 mL) solution of methyl (3R,6R)-1-[4-fluoro-2-(2H-1,2,3-triazol-2-yl)benzoyl]-6-methylpiperidine-3-carboxylate (73.0 mg, 0.21 mmol) obtained in Reference Example 45, and the mixture was stirred at 80°C for 6 hours. The reaction mixture was concentrated under reduced pressure to obtain the title compound (colorless amorphous form).
MS (ESI pos.) m/z : 333 [M+H]+

### Reference Example 47 (3R,6R)-N-(5-Chloro-2-hydroxypyridin-3-yl)-1-[4-fluoro-2-(2H-1,2,3-triazol-2-yl)benzoyl]-6-methylpiperidine-3-carboxamide

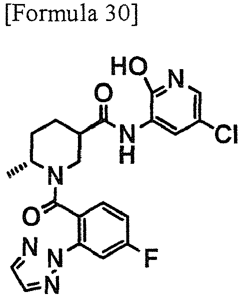

The title compound (25.5 mg) was obtained (brown oil) by the same approach as in Reference Example 44 using (3R,6R)-6-methyl-1-[5-methyl-2-(2H-1,2,3-triazol-2-yl)benzoyl]piperidine-3-carboxylic acid (0.21 mmol) obtained in Reference Example 46 and 3-amino-5-chloro-2-hydroxypyridine hydrochloride (41.82 mg, 0.23 mmol) as starting materials. MS (ESI pos.) m/z : 459 [M+H]+

### Reference Example 48 5-Chloro-2-[(3R,6R)-6-methylpiperidin-3-yl]-1,3-benzoxazole

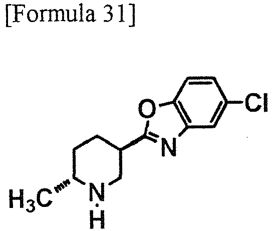

The title compound (2.49 g) was obtained (pale yellow solid) through sequential reactions by the same approach as in Reference Examples 16, 24, and 32 using (3R,6R)-1-(tert-butoxycarbonyl)-6-methylpiperidine-3-carboxylic acid (5.34 g, 21.95 mmol) as a starting material.
MS (ESI pos.) m/z : 251 [M+H]+

### Example 1 [(2R,5R)-5-(5-Chloro-1,3-benzoxazol-2-yl)-2-methylpiperidin-1-yl][6-methyl-3-(2H-1,2,3-triazol-2-yl)pyridin-2-yl]methanone

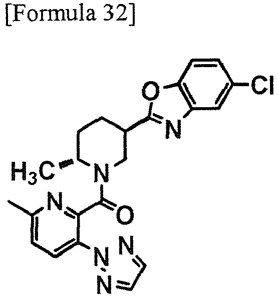

DIPEA (0.228 mL, 1.31 mmol), 6-methyl-3-(2H-1,2,3-triazol-2-yl)pyridine-2-carboxylic acid (66.9 mg, 0.33 mmol), and propane phosphonic acid anhydride (1.7 mol/L EtOAc solution, 0.93 mL, 1.57 mmol) were added to a CHCl₃ solution (5 mL) of 5-chloro-2-[(3RS,6RS)-6-methylpiperidin-3-yl]-1,3-benzoxazole (0.30 g, 1.6 mmol), and the mixture was stirred at 50°C for 4.5 hours. The reaction mixture was concentrated under reduced pressure, and a 2 mol/L aqueous NaOH solution was added to the residue, followed by extraction using EtOAc. The obtained organic layer was dried over MgSO₄, and the desiccant was then filtered off. The solvent was distilled off under reduced pressure. The obtained residue was purified by column chromatography (HP-Sil 10 g, hexane/EtOAc) to obtain a racemic mixture (163 mg) of the title compound (colorless solid).

The obtained racemic mixture was resolved under the above racemic resolution conditions (Condition 2) to separate two peaks (retention time under the analysis conditions: 5.67 min and 8.58 min). Of them, the title compound was obtained (colorless solid) as a compound having a longer relative retention time (retention time: 8.58 min). The absolute stereostructure of the obtained title compound was confirmed because of having the same retention time in the above chiral analysis (Condition A) as that of [(2R,5R)-5-(5-chloro-1,3-benzoxazol-2-yl)-2-methylpiperidin-1-yl][6-methyl-3-(2H-1,2,3-triazol-2-yl)pyridin-2-yl]methanone whose absolute stereostructure was determined after conversion in Reference Example 14.
LCMS retention time 3.80 min. (Condition 3)
MS (ESI pos.) m/z : 437 [M+H]+

### Example 2 [(2R*,5R*)-5-(5-Chloro-1,3-benzoxazol-2-yl)-2-methylpiperidin-1-yl][5-fluoro-2-(2H-1,2,3-triazol-2-yl)phenyl]methanone

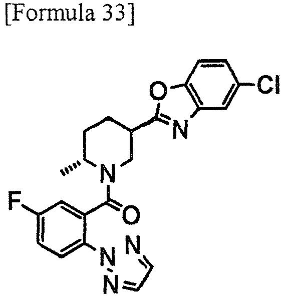

A racemic mixture (34 mg) of the title compound was obtained (colorless solid) by the same approach as in Example 1 using 5-chloro-2-[(3RS,6RS)-6-methylpiperidin-3-yl]-1,3-benzoxazole (50.0 mg, 0.20 mmol) and 5-fluoro-2-(2H-1,2,3-triazol-2-yl)benzoic acid (82.6 mg, 0.40 mmol) as starting materials.

The obtained racemic mixture was resolved under the above racemic resolution conditions (Condition 1). The title compound was obtained (colorless solid) as a compound having a longer relative retention time.
LCMS retention time 4.28 min. (Condition 3)
MS (ESI pos.) m/z : 440 [M+H]+

### Example 3 [(2R*,5R*)-5-(5-Chloro-1,3-benzoxazol-2-yl)-2-methylpiperidin-1-yl][2-(2H-1,2,3-triazol-2-yl)phenyl]methanone

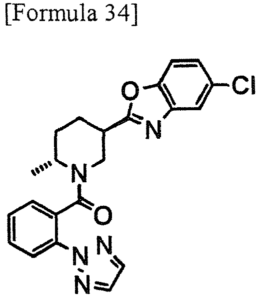

A racemic mixture (37 mg) of the title compound was obtained (colorless solid) by the same approach as in Example 1 using 5-chloro-2-[(3RS,6RS)-6-methylpiperidin-3-yl]-1,3-benzoxazole (32.8 mg, 0.13 mmol) and 2-(2H-1,2,3-triazol-2-yl)benzoic acid (49.5 mg, 0.26 mmol) as starting materials.

The obtained racemic mixture was resolved under the above racemic resolution conditions (Condition 1). The title compound was obtained (colorless solid) as a compound having a longer relative retention time.
LCMS retention time 4.12 min. (Condition 3)
MS (ESI pos.) m/z : 422 [M+H]+

### Example 4 [(2R*,5R*)-5-(5-Chloro-1,3-benzoxazol-2-yl)-2-methylpiperidin-1-yl][6-methyl-3-(pyrimidin-2-yl)pyridin-2-yl]methanone

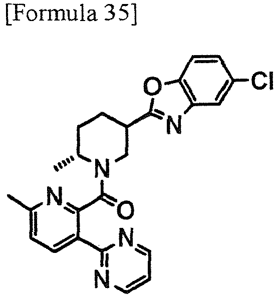

A racemic mixture (58 mg) of the title compound was obtained (colorless solid) by the same approach as in Example 1 using 5-chloro-2-[(3RS,6RS)-6-methylpiperidin-3-yl]-1,3-benzoxazole (98.8 mg, 0.39 mmol) and 6-methyl-3-(pyrimidin-2-yl)pyridine-2-carboxylic acid (84.8 mg, 0.39 mmol) as starting materials.

The obtained racemic mixture was resolved under the above racemic resolution conditions (Condition 2). The title compound was obtained (colorless solid) as a compound having a longer relative retention time.
LCMS retention time 3.52 min. (Condition 3)
MS (ESI pos.) m/z : 448 [M+H]+

### Example 5 [(2R*,5R*)-5-(5-Chloro-1,3-benzoxazol-2-yl)-2-methylpiperidin-1-yl][5-methyl-2-(pyrimidin-2-yl)phenyl]methanone

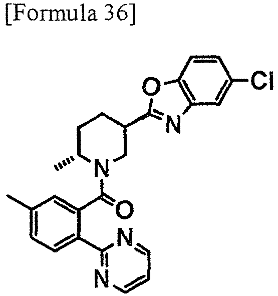

A racemic mixture (84 mg) of the title compound was obtained (colorless solid) by the same approach as in Example 1 using 5-chloro-2-[(3RS,6RS)-6-methylpiperidin-3-yl]-1,3-benzoxazole (55.0 mg, 0.22 mmol) and 5-methyl-2-(pyrimidin-2-yl)benzoic acid (94.0 mg, 0.44 mmol) as starting materials.

The obtained racemic mixture was resolved under the above racemic resolution conditions (Condition 1). The title compound was obtained (colorless solid) as a compound having a longer relative retention time.
LCMS retention time 4.20 min. (Condition 3)
MS (ESI pos.) m/z : 447 [M+H]+

### Example 6 [(2R*,5R*)-5-(5-Chloro-1,3-benzoxazol-2-yl)-2-methylpiperidin-1-yl][5-fluoro-2-(pyrimidin-2-yl)phenyl]methanone

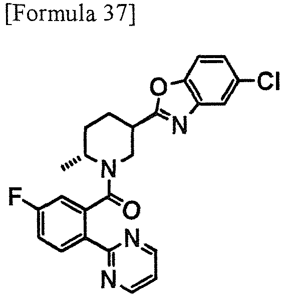

A racemic mixture (66 mg) of the title compound was obtained (colorless solid) by the same approach as in Example 1 using 5-chloro-2-[(3RS,6RS)-6-methylpiperidin-3-yl]-1,3-benzoxazole (50.0 mg, 0.20 mmol) and 5-fluoro-2-(pyrimidin-2-yl)benzoic acid (87.0 mg, 0.40 mmol) as starting materials.

The obtained racemic mixture was resolved under the above racemic resolution conditions (Condition 1). The title compound was obtained (colorless solid) as a compound having a longer relative retention time.
LCMS retention time 4.19 min. (Condition 3)
MS (ESI pos.) m/z : 451 [M+H]+

### Example 7 [(2R*,5R*)-5-(5-Chloro-1,3-benzoxazol-2-yl)-2-methylpiperidin-1-yl][2-(pyrimidin-2-yl)phenyl]methanone

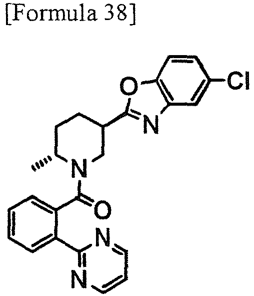

A racemic mixture (83 mg) of the title compound was obtained (colorless solid) by the same approach as in Example 1 using 5-chloro-2-[(3RS,6RS)-6-methylpiperidin-3-yl]-1,3-benzoxazole (50.0 mg, 0.20 mmol) and 2-(pyrimidin-2-yl)benzoic acid (79.8 mg, 0.40 mmol) as starting materials.

The obtained racemic mixture was resolved under the above racemic resolution conditions (Condition 1). The title compound was obtained (colorless solid) as a compound having a longer relative retention time.
LCMS retention time 3.98 min. (Condition 3)
MS (ESI pos.) m/z : 433 [M+H]+

### Example 8 [(2R*,5R*)-5-(5-Fluoro-1,3-benzoxazol-2-yl)-2-methylpiperidin-1-yl][5-methyl-2-(pyrimidin-2-yl)phenyl]methanone

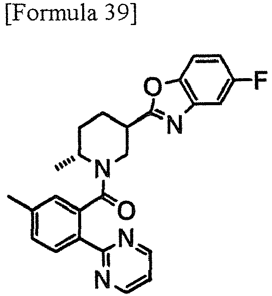

A racemic mixture (55 mg) of the title compound was obtained (colorless solid) by the same approach as in Example 1 using 5-fluoro-2-[(3RS,6RS)-6-methylpiperidin-3-yl]-1,3-benzoxazole (53.0 mg, 0.23 mmol) and 5-methyl-2-(pyrimidin-2-yl)benzoic acid (96.9 mg, 0.45 mmol) as starting materials.

The obtained racemic mixture was resolved under the above racemic resolution conditions (Condition 1). The title compound was obtained (colorless solid) as a compound having a longer relative retention time.
LCMS retention time 3.93 min. (Condition 3)
MS (ESI pos.) m/z : 431 [M+H]+

### Example 9 [(2R*,5R*)-5-(5-Chloro-1,3-benzoxazol-2-yl)-2-methylpiperidin-1-yl][5-methyl-2-(2H-1,2,3-triazol-2-yl)phenyl]methanone

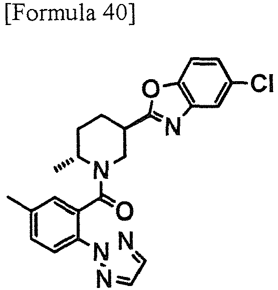

A racemic mixture (65 mg) of the title compound was obtained (colorless solid) by the same approach as in Reference Example 2 using (3RS,6RS)-N-(5-chloro-2-hydroxyphenyl)-6-methyl-1-[5-methyl-2-(2H-1,2,3-triazol-2-yl)benzoyl]piperidine-3-carboxamide (118 mg, 0.26 mmol) obtained in Reference Example 10 as a starting material.

The obtained racemic mixture was resolved under the above racemic resolution conditions (Condition 1). The title compound was obtained (colorless solid) as a compound having a longer relative retention time.
LCMS retention time 4.32 min. (Condition 3)
MS (ESI pos.) m/z : 436 [M+H]+

### Example 10 [(2R*,5R*)-5-(5-Fluoro-1,3-benzoxazol-2-yl)-2-methylpiperidin-1-yl][5-methyl-2-(2H-1,2,3-triazol-2-yl)phenyl]methanone

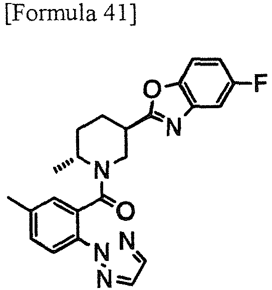

A racemic mixture (65 mg) of the title compound was obtained (colorless solid) by the same approach as in Reference Example 2 using (3RS,6RS)-N-(5-fluoro-2-hydroxyphenyl)-6-methyl-1-[5-methyl-2-(2H-1,2,3-triazol-2-yl)benzoyl]piperidine-3-carboxamide (18 mg, 0.04 mmol) obtained in Reference Example 11 as a starting material.

The obtained racemic mixture was resolved under the above racemic resolution conditions (Condition 1). The title compound was obtained (colorless solid) as a compound having a longer relative retention time.
LCMS retention time 4.05 min. (Condition 3)
MS (ESI pos.) m/z : 420 [M+H]+

### Example 11 [(2R*,5R*)-5-(5-Fluoro-1,3-benzoxazol-2-yl)-2-methylpiperidin-1-yl][6-methyl-3-(2H-1,2,3-triazol-2-yl)pyridin-2-yl]methanone

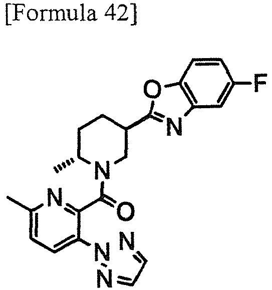

A racemic mixture (55 mg) of the title compound was obtained (colorless solid) by the same approach as in Example 1 using 5-fluoro-2-[(3RS,6RS)-6-methylpiperidin-3-yl]-1,3-benzoxazole (53.0 mg, 0.23 mmol) and 6-methyl-3-(2H-1,2,3-triazol-2-yl)pyridine-2-carboxylic acid (96.9 mg, 0.45 mmol) as starting materials.

The obtained racemic mixture was resolved under the above racemic resolution conditions (Condition 2). The title compound was obtained (colorless solid) as a compound having a longer relative retention time.
LCMS retention time 3.38 min. (Condition 3)
MS (ESI pos.) m/z : 421 [M+H]+

### Example 12 [(2R*,5R*)-5-(6-Fluoro-1,3-benzoxazol-2-yl)-2-methylpiperidin-1-yl][6-methyl-3-(2H-1,2,3-triazol-2-yl)pyridin-2-yl]methanone

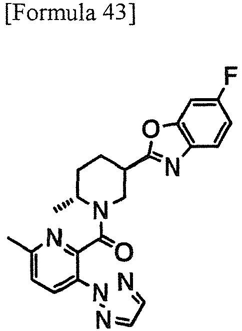

6-Methyl-3-(2H-1,2,3-triazol-2-yl)pyridine-2-carboxylic acid (73.0 mg, 0.36 mmol), HOBt·H₂O (74.0 mg, 0.49 mmol), and EDC·HCl (93.0 mg, 0.49 mmol) were added to a DMF solution (3 mL) of 6-fluoro-2-[(3RS,6RS)-6-methylpiperidin-3-yl]-1,3-benzoxazole hydrochloride (88.0 mg, 0.33 mmol) and TEA (0.05 mL, 0.36 mmol), and the mixture was stirred at room temperature for 3 hours. An aqueous NaHCO₃ solution was added to the reaction solution, followed by extraction using EtOAc. The organic layer was dried over MgSO₄, and the desiccant was then filtered off. The solvent was distilled off under reduced pressure. The obtained residue was purified by column chromatography (HP-Sil 25 g, hexane/EtOAc) to obtain a racemic mixture (32 mg) of the title compound (colorless solid).

The obtained racemic mixture was resolved under the above racemic resolution conditions (Condition 2). The title compound was obtained (colorless solid) as a compound having a longer relative retention time.
LCMS retention time 3.36 min. (Condition 3)
MS (ESI pos.) m/z : 421 [M+H]+

### Example 13 [(2R,5R)-5-(5-Chloro-1,3-benzoxazol-2-yl)-2-methylpiperidin-1-yl][5-chloro-2-(pyrimidin-2-yl)phenyl]methanone

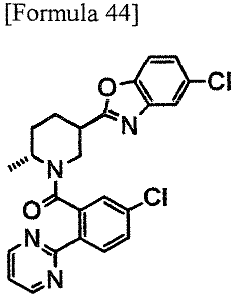

DIPEA (34.7 µL, 0.20 mmol) and propane phosphonic acid anhydride (1.7 mmol/L EtOAc solution, 141 µL, 0.24 mmol) were added to a CHCl₃ solution (1 mL) of 5-chloro-2-[(3R,6R)-6-methylpiperidin-3-yl]-1,3-benzoxazole (20 mg, 0.080 mmol) and 5-chloro-2-(pyrimidin-2-yl)benzoic acid (19 mg, 0.080 mmol), and the mixture was stirred at 50°C for 9 hours. An aqueous NaHCO₃ solution was added to the reaction solution, followed by extraction using CHCl₃. The reaction mixture was concentrated under reduced pressure. Then, the residue was purified by HPLC to obtain the title compound (4.9 mg) (yellow solid).
LCMS retention time 1.16 min. (Condition 4)
MS (ESI pos.) m/z : 467 [M+H]+

The following compounds of Examples 14 to 70 were obtained by the same approach as in Example 13. The structural formulas, names, and LCMS data of the obtained compounds are shown in Tables 5-1 to 5-10.

**[Table 5-1]**

| Example No | Structural formula | Compound name | MS (ESI pos.) m/z | LCMS measurement condition Retention time (min) |
|---|---|---|---|---|
| Example14 | 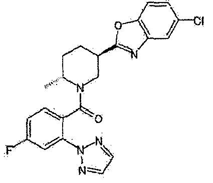 | [(2R,5R)-5-(5-Chloro-1,3-benzoxazol-2-yl)-2-methylpiperidin -1-yl][4-fluoro-2-(2H-1,2,3-triazol -2-yl)phenyl]methanone | 440(M+H)+ | Condition 4 1.10 |
| Example 15 | 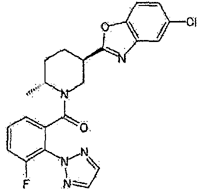 | [(2R,5R)-5-(5-Chloro-1,3-benzoxazol-2-yl)-2-methylpiperidin -1-yl][3-fluoro-2-(2H-1,2,3-triazol -2-yl)phenyl]methanone | 440(M+H)+ | Condition4 1.04 |
| Example16 | 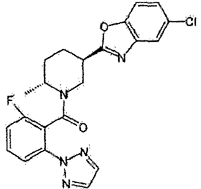 | [(2R,5R)-5-(5-Chloro-1,3-benzoxazol-2-yl)-2-methylpiperidin -1-yl][2-fluoro-6-(2H-1,2,3-triazol -2-yl)phenyl]methanone | 440(M+H)+ | Condition4 1.06 |
| Example17 | 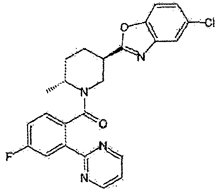 | [(2R,5R)-5-(5-Chloro-1,3-benzoxazol-2-yl)-2-methylpiperidin -1-yl][4-fluoro-2-(pyrimidin-2-yl) phenyl]methanone | 451(M+H)+ | Condition4 1.08 |
| Example18 | 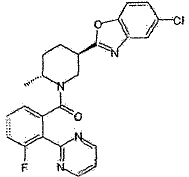 | [(2R,5R)-5-(5-Chloro-1,3-benzoxazol-2-yl)-2-methylpiperidin -1-yl][3-fluoro-2-(pyrimidin-2-yl) phenyl]methanone | 451(M+H)+ | Condition4 0.99 |

**[Table 5-2]**

| Example No | Structural formula | Compound name | MS (ESI pos.) m/z | LCMS measurement condition Retention time (min) |
|---|---|---|---|---|
| Example19 | 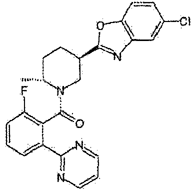 | [(2R,5R)-5-(5-Chloro-1,3-benzoxazol-2-yl)-2-methylpiperidin -1-yl][2-fluoro-6-(pyrimidin-2-yl) phenyl]methanone | 451 (M+H)+ | Condition4 1.00, 1.04 |
| Example20 | 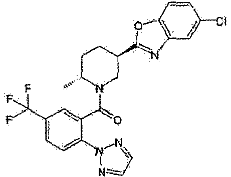 | [(2R,5R)-5-(5-Chloro-1,3-benzoxazol-2-yl)-2-methylpiperidin -1-yl][2-(2H-1,2,3-triazol-2-yl)-5-(trifluoromethyl)phenyl]methanone | 490(M+H)+ | Condition4 1.20 |
| Example21 | 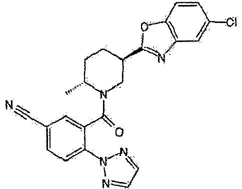 | 3-{[(2R,5R)-5-(5-Chloro-1,3-benzoxazol-2-yl)-2-methylpiperidin -1-yl]carbonyl}-4-(2H-1,2,3-triazol -2-yl)benzonitrile | 447(M+H)+ | Condition2 2.17 |
| Example22 | 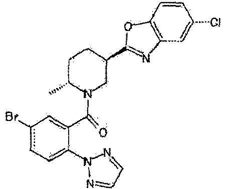 | [5-Bromo-2-(2H-1,2,3-triazol-2-yl) phenyyl][(2R,5R)-5-(5-chloro-1,3-benzoxazol-2-yl)-2-methylpiperidin -1-yl]methanone | 500(M+H)+ | Condition4 1.20 |
| Example23 | 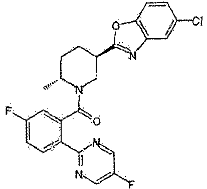 | [(2R,5R)-5-(5-Chloro-1,3-benzoxazol-2-yl)-2-methylpiperidin -1-yl][5-fluoro-2-(5-fluoropyrimidin -2-yl)phenyl]methanone | 469(M+H)+ | Condition4 1.13 |
| Example24 | 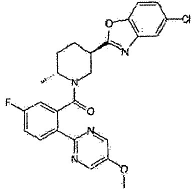 | [(2R,5R)-5-(5-Chloro-1,3-benzoxazol-2-yl)-2-methylpiperidin -1-yl][5-fluoro-2-(5-methoxypyrimidin-2-yl)phenyl] methanone | 481 (M+H)+ | Condition4 1.11 |

**[Table 5-3]**

| Example No | Structural formula | Compound name | MS (ESI pos.) m/z | LCMS measurement condition Retention time (min) |
|---|---|---|---|---|
| Example25 | 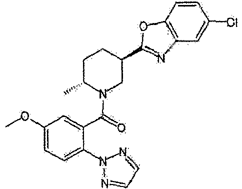 | [(2R,5R)-5-(5-Chloro-1,3-benzoxazol-2-yl)-2-methylpiperidin -1-yl][5-methoxy-2-(2H-1,2,3-triazol-2-yl)phenyl]methanone | 452(M+H)+ | Condition4 0.96 |
| Example26 | 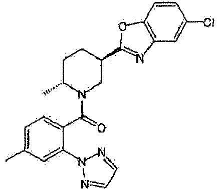 | [(2R,5R)-5-(5-Chloro-1,3-benzoxazol-2-yl)-2-methylpiperidin -1-yl][4-methyl-2-(2H-1,2,3-triazol-2-yl)phenyl]methanone | 436(M+H)+ | Condition4 1.13 |
| Example27 | 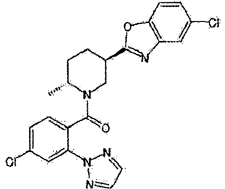 | [(2R,5R)-5-(5-Chloro-1,3-benzoxazol-2-yl)-2-methylpiperidin -1-yl][4-chloro-2-(2H-1,2,3-triazol-2-yl)phenyl]methanone | 456(M+H)+ | Condition4 1.06 |
| Example28 | 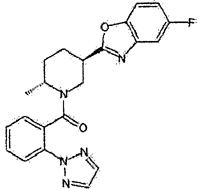 | [(2R,5R)-5-(5-Fluoro-1,3-benzoxazol-2-yl)-2-methylpiperidin -1-yl][2-(2H-1,2,3-triazol-2-yl) phenyl]methanone | 406(M+H)+ | Condition4 0.97 |
| Example29 | 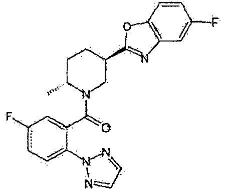 | [(2R,5R)-5-(5-Fluoro-1,3-benzoxazol-2-yl)-2-methylpiperidin -1-yl][5-fluoro-2-(2H-1,2,3-triazol -2-yl)phenyl]methanone. | 424(M+H)+ | Condition4 1.02 |
| Example30 | 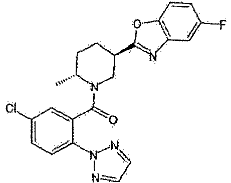 | [5-Chloro-2-(2H-1,2,3-triazol-2-yl) phenyl][(2R,5R)-5-(5-fluoro-1,3-benzoxazol-2-yl)-2-methylpiperidin -1-yl]methanone | 440(M+H)+ | Condition4 1.09 |

**[Table 5-4]**

| Example No | Structural formula | Compound name | MS (ESI pos.)^{.} m/z | LCMS measurement condition Retention time (min) |
|---|---|---|---|---|
| Example31 | 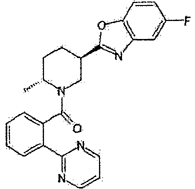 | [(2R,5R)-5-(5-Fluoro-1,3-benzoxazol-2-yl)-2-methylpiperidin -1-yl][2-(pyrimidin-2-yl)phenyl] methanone | 417(M+H)+ | Condition4 0.94 |
| Example32 | 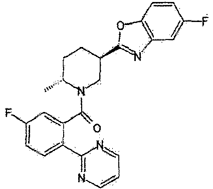 | [(2R,5R)-5-(5-Fluoro-1,3-benzoxazol-2-yl)-2-methylpiperidin -1-yl][5-fluoro-2-(pyrimidin-2-yl) phenyl]methanone | 435(M+H)+ | Condition4 0.99 |
| Example33 | 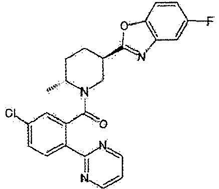 | [5-Choro-2-(pyrimidin-2-yl)phenyl] [(2R,5R)-5-(5-fluoro-1,3-benzoxazol -2-yl)-2-methylpiperidin-1-yl] methanone | 451(M+H)+ | Condition4 1.07 |
| Example34 | 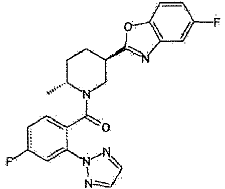 | [(2R,5R)-5-(5-Fluoro-1,3-benzoxazol-2-yl)-2-methylpiperidin -1-yl][4-fluoro-2-(2H-1,2,3-triazol -2-yl)phenyl]methanone | 424(M+H)+ | Condition4 1.02 |
| Example35 | 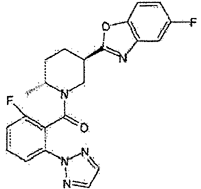 | [(2R,5R)-5-(5-Fluoro-1,3-benzoxazol-2-yl)-2-methylpiperidin -1-yl][2-fluoro-6-(2H-1,2,3-triazol -2-yl)phenyl]methanone | 424(M+H)+ | Condition4 0.98 |
| Example36 | 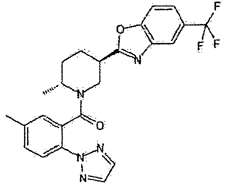 | [5-Methyl-2-(2H-1,2,3-triazol-2-yl) phenyl]{(2R,5R)-2-methyl-5-[5'-(trifluoromethyl)-1,3-benzoxazol-2-yl]piperidin-1-yl}methanone | 470(M+H)+ | Condition4 1:14 |

**[Table 5-5]**

| Example No | Structural formula | Compound name | MS (ESI pos.) m/z | LCMS measurement condition Retention time (min) |
|---|---|---|---|---|
| Example37 | 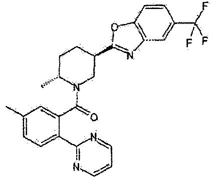 | [5-Methyl-2-(pyrimidin-2-yl)phenyl] [(2R,5R)-2-methyl-5-[5-(trifluoromethyl)-1,3-benzoxazol-2-yl]piperidin-1-yl]methanone | 481(M+H)+ | Condition4 1.11 |
| Example38 | 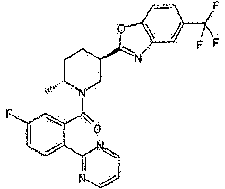 | [5-Fluoro-2-(pyrimidin-2-yl)phenyl] {(2R,5R)-2-methyl-5-[5-(trifluoromethyl)-1,3-benzoxazol-2-yl]piperidin-1-yl}methanone | 485(M+H)+ | Condition4 1.10 |
| Example39 | 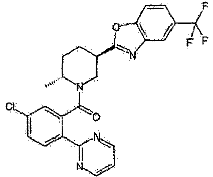 | [5-Chloro-2-(pyrimidin-2-yl)phenyl] [(2R,5R)-2-methyl-5-[5-(trifluoromethyl)-1,3-benzoxazol-2-yl]piperidin-1-yl]methanone | 501(M+H)+ | Condition4 1.17 |
| Example40 | 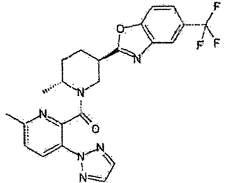 | [6-Methyl-3-(2H-1,2,3-triazol-2-yl) pyridin-2-yl][(2R,5R)-2-methyl-5-[5-(trifluoromethyl)-1,3-benzoxazol -2-yl]piperidin-1-yl]methanone | 471(M+H)+ | Condition4 1.02 |
| Example41 | 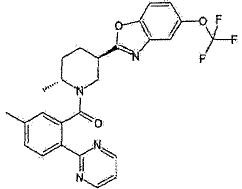 | [5-Methyl-2-(pyrimidin-2-yl)phenyl] [(2R,5R)-2-methyl-5-[5-(trifluoromethoxy)-1,3-benzoxazol -2-yl]piperidin-1-yl]methanone | 497(M+H)+ | Condition4 1.14 |
| Example42 | 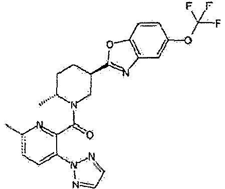 | [6-Methyl-3-(2H-1,2,3-triazol-2-yl)pyridin-2-yl]{(2R,5R)-2-methyl-5-[5-(trifluoromethoxy)-1,3-benzoxazol-2-yl]piperidin-1-yl} methanone | 487(M+H)+ | Condition4 1.04 |

**[Table 5-6]**

| Example No | Structural formula | Compound name | MS (ESI pos.) m/z | LCMS measurement condition Retention time (min) |
|---|---|---|---|---|
| Example43 | 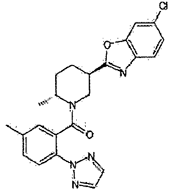 | [(2R,5R)-5-(6-Chloro-1,3-benzoxazol-2-yl)-2-methylpiperidin -1-yl][5-methyl-2-(2H-1,2,3-triazol -2-yl)phenyl]methanone | 436(M+H)+ | Condition4 1.12 |
| Example44 | 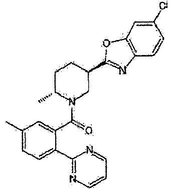 | [(2R,5R)-5-(6-Chloro-1,3-benzoxazol-2-yl)-2-methylpiperidin -1-yl][5-methyl-2-(pyrimidin-2-yl) phenyl]methanone | 447(M+H)+ | Condition4 1.09 |
| Example45 | 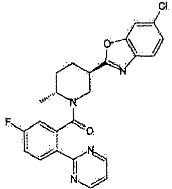 | [(2R,5R)-5-(6-Chloro-1,3-benzoxazol-2-yl)-2-methylpiperidin -1-yl][5-fluoro-2-(pyrimidin-2-yl) phenyl]methanone | 451(M+H)+ | Condition4 1.08 |
| Example46 | 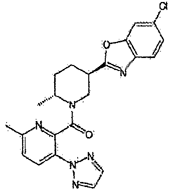 | [(2R,5R)-5-(6-Chloro-1,3-benzoxazol-2-yl)-2-methylpiperidin -1-yl][6-methyl-3-(2H-1,2,3-triazol -2-yl)pyridin-2-yl]methanone | 437(M+H)+ | Condition4 0.97 |
| Example47 | 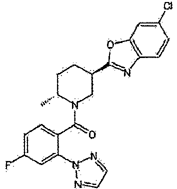 | [(2R,5R)-5-(6-Chloro-1,3-benzoxazol-2-yl)-2-methylpiperidin -1-yl][4-fluoro-2-(2H-1,2,3-triazol -2-yl)phenyl]methanone | 440(M+H)+ | Condition4 1.10 |
| Example48 | 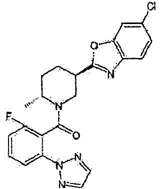 | [(2R,5R)-5-(6-Chloro-1,3-benzoxazol-2-yl)-2-methylpiperidin -1-yl][2-fluoro-6-(2H-1,2,3-triazol -2-yl)phenyl]methanone | 440(M+H)+ | Condition4 1.06 |

**[Table 5-7]**

| Example No | Structural formula | Compound name | MS (ESI pos.) m/z | LCMS measurement condition Retention time (min) |
|---|---|---|---|---|
| Example49 | 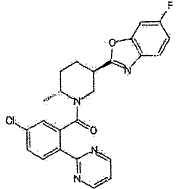 | [5-Chloro-2-(pyrimidin-2-yl)phenyl] [(2R,5R)-5-(6-fluoro-1,3-benzoxazol -2-yl)-2-methylpiperidin-1-yl] methanone | 451(M+H)+ | Condition4 1.07 |
| Example50 | 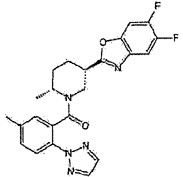 | [(2R,5R)-5-(5,6-Difluoro-1,3-benzoxazol-2-yl)-2-methylpiperidin -1-yl][5-methyl-2-(2H-1,2,3-triazol -2-yl)phenyl]methanone | 438(M+H)+ | Condition4 1.06 |
| Example51 | 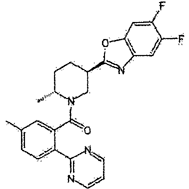 | [(2R,5R)-5-(5,6-Difluoro-1,3-benzoxazol-2-yl)-2-methylpiperidin -1-yl][5-methyl-2-(pyrimidin-2-yl) phenyl]methanone | 449(M+H)+ | Condition4 1.04 |
| Example52 | 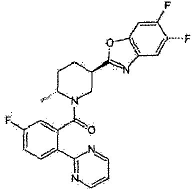 | [(2R,5R)-5-(5,6-Difluoro-1,3-benzoxazol-2-yl)-2-methylpiperidin -1-yl][5-fluoro-2-(pyrimidin-2-yl) phenyl]methanone | 453(M+H)+ | Condition4 1.03 |
| Example53 | 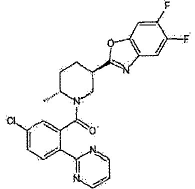 | [5-Chloro-2-(pyrimidin-2-yl)phenyl] [(2R,5R)-5-(5,6-difluoro-1,3-benzoxazol-2-yl)-2-methylpiperidin -1-yl]methanone | 469(M+H)+ | Condition4 1.10 |
| Example54 | 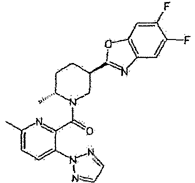 | [(2R,5R)-5-(5,6-Difluoro-1,3-benzoxazol-2-yl)-2-methylpiperidin -1-yl][6-methyl-3-(2H-1,2,3,-triazol -2-yl)pyridin-2-yl]methanone | 439(M+H)+ | Condition4 0.92 |

**[Table 5-8]**

| Example No | Structural formula | Compound name | MS (ESI pos.) m/z | LCMS measurement condition Retention time (min) |
|---|---|---|---|---|
| Example55 | 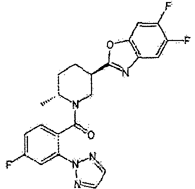 | [(2R,5R)-5-(5,6-Difluoro-1,3-benzoxazol-2-yl)-2-methylpiperidin -1-yl][4-fluoro-2-(2H-1,2,3-triazol -2-yl)phenyl]methanone | 442(M+H)+ | Condition4 1.05 |
| Example56 | 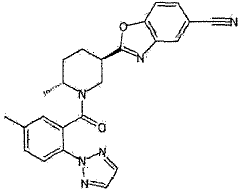 | 2-[(3R,6R)-6-Methyl-1-[5-methyl -2-(2H-1,2,3-triazol-2-yl)benzoyl] piperidin-3-yl]-1,3-benzoxazole-5-carbonitrile | 427(M+H)+ | Condition4 0.96 |
| Example57 | 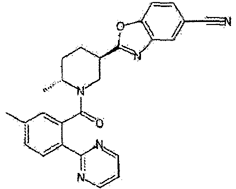 | 2-[(3R,6R)-6-Methyl-1-[5-methyl -2-(pyrimidin-2-yl)benzoyl]piperidin -3-yl]-1,3-benzoxazole-5-carbonitrile | 438(M+H)+ | Condition4 0.93 |
| Example58 | 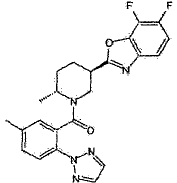 | [(2R,5R)-5-(6,7-Difluoro-1,3-benzoxazol-2-yl)-2-methylpiperidin -1-yl][5-methyl-2-(2H-1,2,3-triazol -2-yl)phenyl]methanone | 438(M+N)+ | Condition4 1.09 |
| Example59 | 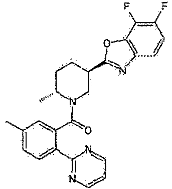 | [(2R,5R)-5-(6.7-Difluoro-1,3-benzoxazol-2-yl)-2-methylpiperidin -1-yl][5-methyl-2-(pyrimidin-2-yl) phenyl]methanone | 449(M+H)+ | Condition4 1.06 |
| Example60 | 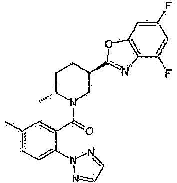 | [(2R,5R)-5-(4,6-Difluoro-1,3-benzoxazol-2-yl)-2-methylpiperidin -1-yl][5-methyl-2-(2H-1,2,3-triazol -2-yl)phenyl]methanone | 460 (M+Na)+ | Condition4 1.08 |

**[Table 5-9]**

| Example No | Structural formula | Compound name | MS (ESI pos.) m/z | LCMS measurement condition Retention time (min) |
|---|---|---|---|---|
| Example61 | 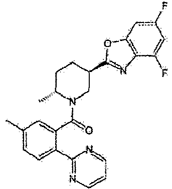 | [(2R,5R)-5-(4,6-Difluoro-1,3-benzoxazol-2-yl)-2-methylpiperidin -1-yl][5-methyl-2-(pyrimidin-2-yl) phenyl]methanone | 449(M+H)+ | Condition4 1.06 |
| Example62 | 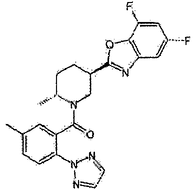 | [(2R,5R)-5-(5,7-Difluoro-1,3-benzoxazol-2-yl)-2-methylpiperidin -1-yl][5-methyl-2-(2H-1,2,3-triazol -2-yl)phenyl]methanone | 438(M+H)+ | Condition4 1.08 |
| Example63 | 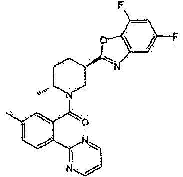 | [(2R,5R)-5-(5,7-Difluoro-1,3-benzoxazol-2-yl)-2-methylpiperidin -1-yl][5-methyl-2-(pyrimidin-2-yl) phenyl]methanone | 449(M+H)+ | Condition4 1.05 |
| Example64 | 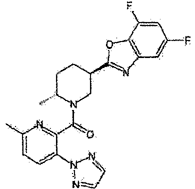 | [(2R,5R)-5-(5,7-Difluoro-1,3-benzoxazol-2-yl)-2-methylpiperidin -1-yl][6-methyl-3-(2H-1,2,3-triazol -2-yl)pyridin-2-yl]methanone | 439(M+H)+ | Condition4 0.94 |
| Example65 | 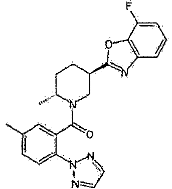 | [(2R,5R)-5-(7-Fluoro-1,3-benzoxazol-2-yl)-2-methylpiperidin -1-yl][5-methyl-2-(2H-1,2,3-triazol -2-yl)phenyl]methanone | 420(M+H)+ | Condition4 1.05 |
| Example66 | 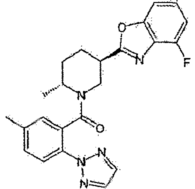 | [(2R,5R)-5-(4-Fluoro-1,3-benzoxazol-2-yl)-2-methylpiperidin -1-yl][5-methyl-2-(2H-1,2,3-triazol -2-yl)phenyl]methanone | 420(M+H)+ | Condition4 1.05 |

**[Table 5-10]**

| Example No | Structural formula | Compound name | MS (ESI pos.) m/z | LCMS measurement condition Retention time (min) |
|---|---|---|---|---|
| Example67 | 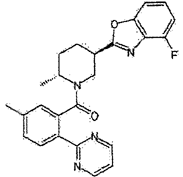 | [(2R,5R)-5-(4-Fluoro-1,3-benzoxazol-2-yl)-2-methylpiperidin -1-yl][5-methyl-2-(pyrimidin-2-yl) phenyl]methanone | 431(M+H)+ | Condition4 1.02 |
| Example68 | 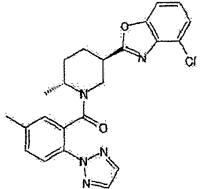 | [(2R,5R)-5-(4-Chloro-1,3-benzoxazol-2-yl)-2-methylpiperidin -1-yl][5-methyl-2-(2H-1,2,3-triazol -2-yl)phenyl]methanone | 436(M+H)+ | Condition4 1.11 |
| Example69 | 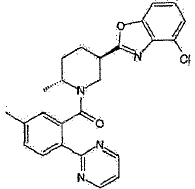 | [(2R,5R)-5-(4-Chloro-1,3-benzoxazol-2-yl)-2-methylpiperidin -1-yl][5-methyl-2-(pyrimidin-2-yl) phenyl]methanone | 447(M+H)+ | Condition4 1.08 |
| Example70 | 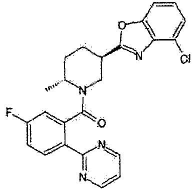 | [(2R,5R)-5-(4-Chloro-1,3-benzoxazol-2-yl)-2-methylpiperidin -1-yl][5-fluoro-2-(pyrimidin-2-yl) phenyl]methanone | 451(M+H)+ | Condition4 1.07 |

### Example 71 [(2R,5R)-5-(6-Chloro[1,3]oxazolo[5,4-b]pyridin-2-yl)-2-methylpiperidin-1-yl][5-methyl-2-(2H-1,2,3-triazol-2-yl)phenyl]methanone

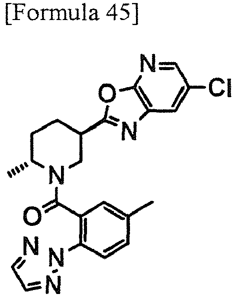

A CHCl₃ solution (2 mL) of (3R,6R)-N-(5-chloro-2-hydroxypyridin-3-yl)-6-methyl-1-[5-methyl-2-(2H-1,2,3-triazol-2-yl)benzoyl]piperidine-3-carboxamide (50 mg, 0.11 mmol), PPh₃ (87 mg, 0.33 mmol), C₂Cl₆ (65 mg, 0.27 mmol), and TEA (120 µL, 0.88 mmol) was stirred overnight at room temperature. After concentration under reduced pressure, the residue was purified by HPLC to obtain the title compound (17 mg) (colorless solid).
LCMS retention time 1.03 min. (Condition 4)
MS (ESI pos.) m/z : 437(M+H)+

### Example 72 [(2R,5R)-5-(6-Chloro[1,3]oxazolo[5,4-b]pyridin-2-yl)-2-methylpiperidin-1-yl][4-fluoro-2-(2H-1,2,3-triazol-2-yl)phenyl]methanone

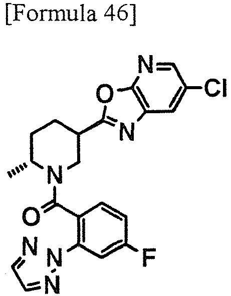

DEAD (2.2 mol/L, 0.05 mL, 0.10 mmol) was added to a THF solution (0.5 mL) of (3R,6R)-N-(5-chloro-2-hydroxypyridin-3-yl)-1-[4-fluoro-2-(2H-1,2,3-triazol-2-yl)benzoyl]-6-methylpiperidine-3-carboxamide (21.40 mg, 0.05 mmol) and PPh₃ (26.91 mg, 0.10 mmol) under ice cooling, and the mixture was stirred overnight at room temperature. PPh₃ (26.91 mg, 0.10 mmol) and DEAD (2.2 mol/L, 0.05 mL, 0.10 mmol) were further added thereto, and the mixture was stirred at 50°C for 2 hours. After concentration under reduced pressure, the residue was purified by HPLC to obtain the title compound (5.2 mg) (colorless solid).
LCMS retention time 1.01 min. (Condition 4)
MS (ESI pos.) m/z : 463 (M+Na)+

### Test Example 1 (Orexin antagonistic activity assay)

The antagonistic activity of each test compound against human orexin-1 receptor (hOX1R) and orexin-2 receptor (hOX2R) was assayed by a modification of a method described in the literature (Toshikatsu Okumura et al., Biochemical and Biophysical Research Communications 280, 976-981, 2001). Chinese hamster ovary (CHO) cells forced to express hOX1R or hOX2R were inoculated at a density of 20,000 cells/well to a 96-well Black clear bottom plate (Nunc) and cultured at 37°C for 16 hours under 5% CO₂ conditions in a Ham's F-12 medium containing 0.1 mM MEM nonessential amino acids, 0.5 mg/ml G418, and 10% fetal bovine serum (all from Invitrogen Corp.). After removal of the medium, 100 µL of a buffer solution for assay (25 mM HEPES (Dojindo Laboratories), Hanks' balanced salt solution (Invitrogen Corp.), 0.1% bovine serum albumin, 2.5 mM probenecid, and 200 µg/ml Amaranth (all from Sigma-Aldrich Corp.), pH 7.4) containing 0.5 µM Fluo-3 AM ester (Dojindo Laboratories) was added to the cells, which were then incubated at 37°C for 60 minutes under 5% CO₂ conditions. After removal of the buffer solution for assay containing Fluo-3 AM ester, each test compound was dissolved at a concentration of 10 mM in dimethyl sulfoxide and diluted with a buffer solution for assay. Then, 150 µL of the solution was added to the cells, which were then incubated for 30 minutes.

A ligand peptide (Pyr-Pro-Leu-Pro-Asp-Ala-Cys-Arg-Gln-Lys-Thr-Ala-Ser-Cys-Arg-Leu-Tyr-Glu-Leu-Leu-His-Gly-Ala-Gly-Asn-His-Ala-Ala-Gly-Ile-Leu-Thr-Leu-NH2; Peptide Institute, Inc.) derived from human orexin-A by the substitution of two amino acids was diluted with a buffer solution for assay to final concentrations of 300 pM for hOX1R and 3 nM for hOX2R. Reaction was initiated by the addition of 50 µL of this ligand solution. The fluorescence intensity of the reaction was measured in each well every second for 3 minutes using Functional Drug Screening System (FDSS; manufactured by Hamamatsu Photonics K.K.), and the maximum fluorescence intensity was used as an index for intracellular Ca²⁺ concentration to determine antagonistic activity. The antagonistic activity of the test compound was calculated with fluorescence intensity from a well supplemented with only a diluting buffer solution as 100% and fluorescence intensity from a well supplemented with a buffer solution free from the ligand and the compound as 0%. The 50% inhibitory concentration (IC₅₀ value) was determined on the basis of fluorescence intensity derived from the test compound added at varying concentrations.

The IC₅₀ values of the compounds of the present invention are shown in Table 6.

**[Table 6]**

| Example No | IC₅₀ value | | Example No | IC₅₀ value | | Example No | IC₅₀ value | |
|---|---|---|---|---|---|---|---|---|
| | OX1 (nM) | OX2 (nM) | | OX1 (nM) | OX2 (nM) | | OX1 (nM) | OX2 (nM) |
| 1 | 11.0 | 12.2 | 25 | 387.1 | 66.0 | 49 | 103.1 | 93.1 |
| 2 | 19.8 | 8.7 | 26 | 11.0 | 8.4 | 50 | 9.8 | 8.6 |
| 3 | 18.1 | 4.1 | 27 | 1792.3 | 186.6 | 51 | 13.5 | 7.3 |
| 4 | 4.3 | 17.2 | 28 | 50.4 | 1.5 | 52 | 82.9 | 11.0 |
| 5 | 3.1 | 4.3 | 29 | 110.9 | 6.0 | 53 | 56.3 | 13.2 |
| 6 | 54.5 | 2.9 | 30 | 74.4 | 3.7 | 54 | 98.7 | 52.0 |
| 7 | 49.5 | 2.0 | 31 | 68.1 | 29.7 | 55 | 207.1 | 9.4 |
| 8 | 0.7 | 2.1 | 32 | 24.9 | 8.2 | 56 | 163.8 | 10.0 |
| 9 | 1.2 | 1.8 | 33 | 44.6 | 9.2 | 57 | 560.9 | 20.1 |
| 10 | 4.8 | 4.9 | 34 | 212.5 | 8.4 | 58 | 9.3 | 9.4 |
| 11 | 42.1 | 43.7 | 35 | 91.1 | 5.0 | 59 | 4.4 | 9.2 |
| 12 | 111.3 | 104.7 | 36 | 8.9 | 2.5 | 60 | 93.3 | 45.7 |
| 13 | 4.4 | 13.0 | 37 | 36.5 | 9.3 | 61 | 81.3 | 44.2 |
| 14 | 41.6 | 8.8 | 38 | 813.6 | 78.6 | 62 | 24.8 | 4.3 |
| 15 | 141.7 | 9.7 | 39 | 122.7 | 26.3 | 63 | 29.1 | 5.5 |
| 16 | 25.6 | 3.6 | 40 | 156.8 | 100.2 | 64 | 144.4 | 28.5 |
| 17 | 54.5 | 10.3 | 41 | 176.9 | 45.1 | 65 | 8.6 | 6.6 |
| 18 | 300.5 | 59.5 | 42 | 1040.9 | 919.2 | 66 | 36.5 | 20.4 |
| 19 | 83.7 | 9.3 | 43 | 4.1 | 9.8 | 67 | 27.7 | 25.9 |
| 20 | 108.2 | 55.7 | 44 | 9.1 | 23.1 | 68 | 7.1 | 4.6 |
| 21 | 211.3 | 155.0 | 45 | 77.2 | 12.2 | 69 | 4.4 | 5.5 |
| 22 | 31.9 | 7.8 | 46 | 10.0 | 70.0 | 70 | 12.3 | 16.2 |
| 23 | 167.8 | 22.5 | 47 | 58.6 | 16.6 | 71 | 106.5 | 6.1 |
| 24 | 99.2 | 8.5 | 48 | 58.8 | 12.4 | 72 | 412.0 | 80.5 |

### Test Example 2 (Metabolic stability test)

The stability test on a test compound was conducted according to the following method using human liver microsomes (Ms):
The test compound was incubated (37°C, 15 minutes) together with human liver microsome fraction (XenoTech LLC; H630B, lot. 0810472) in a 0.1 M phosphate buffer solution (pH 7.4) in the presence of a NADPH production system (0.16 mM NADP+, 2.5 mM MgCl₂, and 1.5 mM glucose-6-phosphate). The final concentrations of the test compound and the liver Ms protein were set to 1 µM and 0.25 mg protein/mL, respectively. After the incubation, the reaction mixture was supplemented with a 2-fold volume of DMSO, stirred, and then centrifuged at 2150 x g (4°C, 10 minutes). The obtained supernatant was analyzed using a liquid chromatography tandem mass spectrometry (LC-MS/MS) system. The lower limit of quantification was 0.1 µM. As a result, the compound of Example 1 had a rate of metabolism of 27.8%.

### Industrial Applicability

The compound of the present invention was shown to have OX receptor antagonist activity. Thus, the compound of the present invention or the pharmaceutically acceptable salt thereof can be used as a therapeutic or prophylactic agent for diseases controlled by OX receptor antagonist activity, for example, sleep disorder, depression, anxiety disorder, panic disorder, schizophrenia, drug dependence, Alzheimer's disease, Parkinson's disease, Huntington's chorea, eating disorder, headache, migraine, pain, gastrointestinal disease, epilepsy, inflammation, immune-related disease, endocrine-related disease, and hypertension.

## Claims

1. A methylpiperidine derivative represented by formula (IA): 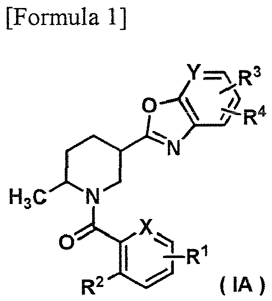 wherein
X represents a nitrogen atom or a formula CH;
Y represents a nitrogen atom or a formula CH;
R¹ represents a hydrogen atom, a halogen atom, a cyano group, a C₁₋₆ alkyl group (wherein the C₁₋₆ alkyl group may be substituted with 1 to 3 halogen atoms), or a C₁₋₆ alkoxy group;
R² represents a heteroaryl group (wherein the heteroaryl group may be substituted with 1 to 3 identical or different substituents selected from the group consisting of a halogen atom and a C₁₋₆ alkoxy group); and
R³ and R⁴ are the same or different and each represent a hydrogen atom, a halogen atom, a cyano group, a C₁₋₆ alkyl group, or a C₁₋₆ alkoxy group (wherein the C₁₋₆ alkyl group and the C₁₋₆ alkoxy group may each be substituted with 1 to 3 halogen atoms)
or a pharmaceutically acceptable salt thereof.

2. The methylpiperidine derivative or the pharmaceutically acceptable salt thereof according to claim 1, wherein in formula (IA),
R³ is a halogen atom, a cyano group, a C₁₋₆ alkyl group, or a C₁₋₆ alkoxy group (wherein the C₁₋₆ alkyl group and the C₁₋₆ alkoxy group may each be substituted with 1 to 3 halogen atoms), and
R⁴ is a hydrogen atom or a halogen atom.

3. The methylpiperidine derivative or the pharmaceutically acceptable salt thereof according to claim 1 or 2, wherein in formula (IA),
R² is a triazolyl group or a pyrimidinyl group (wherein the pyrimidinyl group may be substituted with 1 to 3 identical or different substituents selected from the group consisting of a halogen atom and a C₁₋₆ alkoxy group).

4. The methylpiperidine derivative or the pharmaceutically acceptable salt thereof according to any one of claims 1 to 3, wherein in formula (IA),
R¹ is a hydrogen atom, a halogen atom, or a C₁₋₆ alkyl group.

5. The methylpiperidine derivative or the pharmaceutically acceptable salt thereof according to any one of claims 1 to 4, wherein in formula (IA),
Y is a formula CH, and
R² is a triazolyl group or a pyrimidinyl group.

6. The methylpiperidine derivative or the pharmaceutically acceptable salt thereof according to any one of claims 1 to 5, wherein in formula (IA),
X is a nitrogen atom, and
R⁴ is a hydrogen atom.

7. The methylpiperidine derivative or the pharmaceutically acceptable salt thereof according to any one of claims 1 to 6, wherein formula (IA) is formula (IIA):

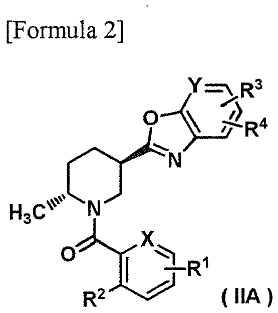

8. A methylpiperidine derivative represented by formula (I): 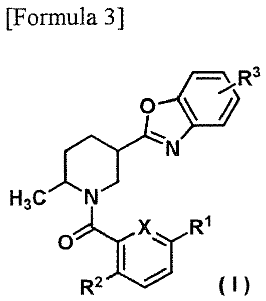 wherein
X represents a nitrogen atom or a formula CH;
R¹ represents a hydrogen atom, a halogen atom, or a C₁₋₆ alkyl group;
R² represents a heteroaryl group (wherein the heteroaryl group may be substituted with 1 to 3 halogen atoms); and
R³ represents a hydrogen atom, a halogen atom, a C₁₋₆ alkyl group, or a C₁₋₆ alkoxy group (wherein the C₁₋₆ alkyl group and the C₁₋₆ alkoxy group may each be substituted with 1 to 3 halogen atoms)
or a pharmaceutically acceptable salt thereof.

9. The methylpiperidine derivative or the pharmaceutically acceptable salt thereof according to claim 8, wherein in formula (I),
X is a nitrogen atom,
R¹ is a hydrogen atom or a C₁₋₆ alkyl group,
R² is a triazolyl group or a pyrimidinyl group, and
R³ is a halogen atom.

10. The methylpiperidine derivative or the pharmaceutically acceptable salt thereof according to claim 8 or 9, wherein formula (I) is formula (II):

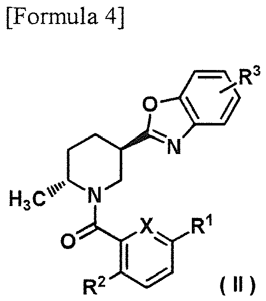

11. A pharmaceutical composition comprising the methylpiperidine derivative or the pharmaceutically acceptable salt thereof according to any one of claims 1 to 10 as an active ingredient.

12. A therapeutic or prophylactic agent for diseases: sleep disorder, depression, anxiety disorder, panic disorder, schizophrenia, drug dependence, Alzheimer's disease, Parkinson's disease, Huntington's chorea, eating disorder, headache, migraine, pain, gastrointestinal disease, epilepsy, inflammation, immune-related disease, endocrine-related disease, and hypertension, comprising the methylpiperidine derivative or the pharmaceutically acceptable salt thereof according to any one of claims 1 to 10 as an active ingredient.
